# EUROPEAN PATENT APPLICATION

(11) **EP 3 028 764 A1**
(43) Date of publication of application: **08.06.2016**
(21) Application number: 14832896.6
(22) Date of filing: 31.07.2014
(51) Int. Cl.: B01L 3/00, C12M 1/00, G01N 21/03, G01N 33/50, G01N 35/02

(54) **PLATE, PRODUCTION METHOD FOR PLATE, BIOCHIP OBSERVATION METHOD, AND SCREENING METHOD**

(30) Priority: 02.08.2013 JP 2013161769
(71) Applicant: Nikon Corporation, Tokyo 108-6290 (JP)
(72) Inventor: ISAMI, Tadao, Tokyo 100-8331 (JP); HAMASHIMA, Muneki, Tokyo 100-8331 (JP); UEDA, Takehiko, Tokyo 100-8331 (JP); HAYASHI, Yutaka, Tokyo 100-8331 (JP)
(74) Representative: Hoffmann Eitle
(86) International application number: PCT/JP2014/070227
(87) International publication number: WO 2015/016315

(57) **Abstract**

This plate (100) is provided with a board (130) having a first surface, and partitioned member (120) in which a plurality of partitions are formed by dividing walls (22). Gaps (28) are formed to the first surface side between neighboring dividing walls (22). The ends of the dividing walls (22) at the gap (28) side have welded portions welded to the first surface of the board (130), the board (130) and the partitioned member (120) being welded at the welded portions.

## Description

### BACKGROUND

### (Technical Field)

The present invention relates to a plate, a method for manufacturing the plate, a method for observing a biochip, and a method for screening.

### (Background)

A microplate where a bottom transparent board that closes an opening side of each cell provided in a plate main body is fastened to a plate main body bottom 1a with an adhesive is well known (for example, see Patent Document 1).

### (Documents of the Related Art)

### (Patent Documents)

Patent Document 1: Japanese Unexamined Patent Application No. 2008-203193

### SUMMARY

### (Problem to Be Solved by the Invention)

There is a risk that plate surface accuracy will drop when a plate is formed using a partitioned member and a board.

### (Means for Solving the Problem)

One mode of the present invention is a plate characterized in that said plate provides a board having a first surface and a partitioned member that forms a plurality of partitions using barrier walls, where a gap, which is a space between adjacent barrier walls, is formed on the first surface side, an end on the gap side of the barrier wall has a welded part that joins with the first surface of the board, and the board and the partitioned member are welded together at the welded part.

Furthermore, another mode of the present invention is characterized in that, in the mode described above, the board and partitioned member are joined together using ultrasonic welding.

Furthermore, another mode of the present invention is characterized in that, in the mode described above, the welded part is welded to the first surface of the board across the entire circumference of the barrier wall.

Furthermore, another mode of the present invention is characterized in that, in the mode described above, the welded part is welded to the first surface of the board on a portion of the circumference of the barrier wall.

Furthermore, another mode of the present invention is characterized in that, in the mode described above, the welded part has a protruding shape, and a sealing member is provided arranged between the partitioned member and the board.

Furthermore, another mode of the present invention is characterized in that, in the mode described above, the protruding welded part passes through the sealing member.

Furthermore, another mode of the present invention is characterized in that, in the mode described above, the board and the partitioned member are formed using the same optically transparent resin material.

Furthermore, another mode of the present invention is characterized in that, in the mode described above, a protective film that covers the open surface of the partition is provided.

Furthermore, another mode of the present invention is characterized in that, in the mode described above, a biochip is provided that is arranged on the bottom of the partition in the first surface.

Furthermore, another mode of the present invention is a method for manufacturing a plate having a plurality of wells formed by joining a partitioned member in a first surface of a board, where a barrier wall of the partitioned member that forms the wells has a gap, which is a space between adjacent barrier walls, on the first surface side of the board, that includes a welding step that welds the first surface of the board and the partitioned member together at a welded part provided on an end on the gap side of the barrier wall.

Furthermore, another mode of the present invention is characterized in that, in the welding step in the mode described above, the welded part of the partitioned member and the first surface of the board are brought into contact and joined by heating past a melting point and then applying pressure.

Furthermore, another mode of the present invention is characterized in that, in the mode described above, the welded part of the partitioned member and the first surface of the board are ultrasonically welded together.

Furthermore, another mode of the present invention is characterized in that a sealing member having a shape that follows the shape of an end on the gap side of the barrier wall on the first surface of the board is provided, and a step that aligns the positions of the sealing member and the partitioned member is included in the mode described above.

Furthermore, another mode of the present invention is characterized in that, in the mode described above, the welded part is welded across the entire circumference of the barrier wall against the first surface of the board.

Furthermore, another mode of the present invention is characterized in that, in the mode described above, the welded part is welded on a portion of the circumference of the barrier wall against the first surface of the board.

Furthermore, another mode of the present invention is characterized in that, in the mode described above, an inserting step that inserts the welded part into the sealing member and then brings the first surface of the board and the welded part of the partitioned member into contact is included.

Furthermore, another mode of the present invention is a method for observing a biochip that includes a step that removes the partitioned member from the plate, and an observing step for observing a biochip arranged on the first surface of the board.

Furthermore, another mode of the present invention is a screening method characterized in that said screening method uses a plate having a biochip, and includes a dispensing step for dispensing a specimen containing a target that is able to react specifically to a biomolecule fixed on the biochip in the plate of the mode described above, a cleaning step for cleaning the plate, a drying step for drying the plate, and a detecting step for detecting affinity between the target and the biomolecule.

Furthermore, another mode of the present invention is a plate characterized in that said plate provides a board, and a regulating member that is joined to the board through welding and regulates a support area in order to support the biochip.

Furthermore, another mode of the present invention is a plate characterized in that said plate has a first regulating member that regulates a first support area in order to support a first biochip where the regulating member is joined to the board through welding and a second regulating member that regulates a second support area in order to support a second biochip, is configured with a body that is separate from the first regulating member, and is joined to the board through welding.

Furthermore, another mode of the present invention is characterized in that, in the mode described above, the length of the first regulating member and the length of the second regulating member are the same in a direction perpendicular to the surface where the board and the regulating members join.

Furthermore, another mode of the present invention is characterized in that, in the mode described above, the regulating member has a gap on the end that joins with the board.

Furthermore, another mode of the present invention is characterized in that, in the mode described above, the support areas regulated by the regulating members are round.

Furthermore, another mode of the present invention is characterized in that, in the mode described above, the board is formed using a material that is translucent.

Furthermore, another mode of the present invention is characterized in that, in the mode described above, the regulating members are formed using a material that is translucent.

Furthermore, another mode of the present invention is characterized in that, in the mode described above, the board is formed from the same material as the regulating members.

Furthermore, another mode of the present invention is characterized in that, in the mode described above, the regulating members are joined to the board using ultrasonic welding.

### BRIEF DESCRIPTION OF THE DRAWINGS

FIG. 1 is a perspective view of the plate according to the first embodiment of the present invention.
FIG. 2 is a bottom plane view of the plate according to the first embodiment of the present invention.
FIG. 3 is a bottom plane view of a partitioned member of the plate according to the first embodiment of the present invention.
FIG. 4 is a cross sectional view of the plate according to the first embodiment of the present invention.
FIG 5 is a cross sectional view of the plate that applies the partitioned member according to the first modified embodiment.
FIG. 6 is a cross sectional view of the plate that applies the partitioned members according to the second modified embodiment.
FIG. 7 is a cross sectional view of the plate that applies the partitioned member according to the third modified embodiment.
FIG. 8 is a bottom surface view and a side view of the partitioned member according to the fourth modified embodiment.
FIG. 9 is a cross sectional view of the plate that applies the partitioned member according to the fourth modified embodiment.
FIG 10 is a flow chart illustrating the procedure for manufacturing the plate that applies the partitioned member according to the fourth modified embodiment.
FIG. 11 is a bottom surface view and a cross sectional view of the plate that applies the partitioned members according to a fifth modified embodiment.
FIG 12 is a cross sectional view of a plate that applies the partitioned member according to the sixth modified embodiment.
FIG. 13 is a side view of a plate that applies the board according to the modified embodiment.
FIG 14 is a cross sectional view of the plate according to the second embodiment of the present invention.
FIG. 15 is a flow chart illustrating the method for producing the plate according to the second embodiment of the present invention.
FIG. 16 is a cross sectional view of the plate according to modified embodiment of the second embodiment of the present invention.
FIG 17 is schematic view of the screening device for executing the screening process for the plate according to the second embodiment of the present invention.
FIG. 18 is a flow chart illustrating the screening method using the screening device.
FIG. 19 is a flow chart illustrating the cleaning and drying step in the cleaning and drying device.

### DETAILED DESCRIPTION

Detailed descriptions of embodiments of the present invention will be given below with reference to the drawings.

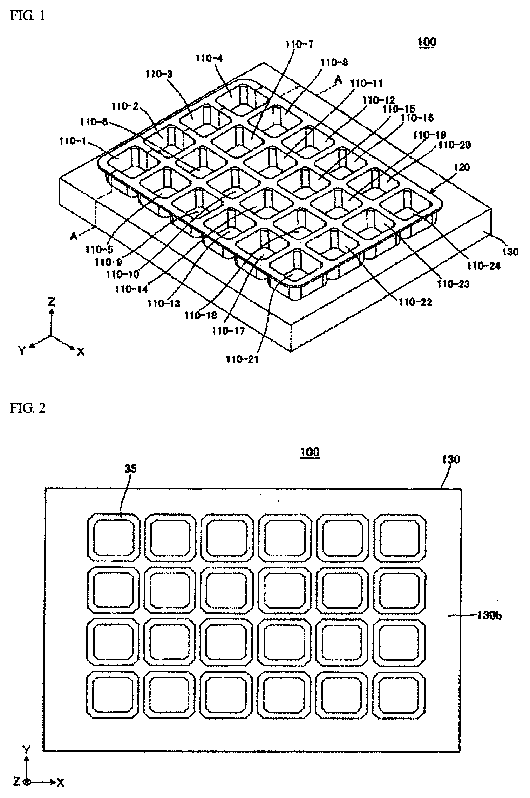

FIG 1 is a perspective view of the plate 100 according to a first embodiment of the present invention. For the convenience of description, the direction perpendicular to the second surface 130b of a board 130 that configures the plate 100 will be the Z axis direction, the direction along the long side of the board 130 that configures the plate 100 will be the X axis direction, and the direction along the short side of the board 130 that configures the plate 100 will be the Y axis direction in the present embodiment. The X axis, Y axis and Z axis cross one another and thus configure intersecting coordinates.

The plate 100 provides the partitioned member 120 and the board 130.

The partitioned member 120 has the first surface 120a and the second surface 120b. The first surface 120a of the partitioned member 120 is a surface that is parallel to the XY plane. The second surface 120b of the partitioned member 120 is a surface that is parallel to the XY plane. The first surface 120a of the partitioned member 120 is formed in a different position in the Z axis direction than the second surface 120b of the partitioned member 120. The first surface 120a of the partitioned member 120 is parallel to the second surface 120b of the partitioned member 120. A partition 20 is a through hole that passes through from the first surface 120a to the second surface 120b. The partition 20 is a space substantially having the shape of a quadrangular prism. However, the shape of the partition 20 is not limited to this and thus may be, for example, the shape of a cylinder.

The partitioned member 120 provides a barrier wall 22, a linking part 24, and a reinforcing part 26.

The barrier wall 22 is a member that forms the partition 20. The partitioned member 120 has the partition 20, which is formed by the barrier wall 22.
As is described below, the partitioned member 120 includes, first barrier wall 22-1, second barrier wall 22-2, third barrier wall 22-3, fourth barrier wall 22-4, fifth barrier wall 22-5, sixth barrier wall 22-6, seventh barrier wall 22-7, eighth barrier wall 22-8, ninth barrier wall 22-9, tenth barrier wall 22-10, eleventh barrier wall 22-11, twelfth barrier wall 22-12, thirteenth barrier wall 22-13, fourteenth barrier wall 22-14, fifteenth barrier wall 22-15, sixteenth barrier wall 22-16, seventeenth barrier wall 22-17, eighteenth barrier wall 22-18, nineteenth barrier wall 22-19, twentieth barrier wall 22-20, twenty first barrier wall 22-21, twenty second barrier wall 22-22, twenty third barrier wall 22-23, and twenty fourth barrier wall 22-24 as barrier wall 22.

Thus, the partitioned member 120 has a total of 24 partitions, partition 20-1 formed by the first barrier wall 22-1, partition 20-2 formed by the second barrier wall 22-2, partition 20-3 formed by the third barrier wall 22-3, partition 20-4 formed by the fourth barrier wall 22-4, partition 20-5 formed by the fifth barrier wall 22-5, partition 20-6 formed by the sixth barrier wall 22-6, partition 20-7 formed by the seventh barrier wall 22-7, partition 20-8 formed by the eighth barrier wall 22-8, partition 20-9 formed by the ninth barrier wall 22-9, partition 20-10 formed by the tenth barrier wall 22-10, partition 20-11 formed by the eleventh barrier wall 22-11, partition 20-12 formed by the twelfth barrier wall 22-12, partition 20-13 formed by the thirteenth barrier wall 22-13, partition 20-14 formed by the fourteenth barrier wall 22-14, partition 20-14 formed by the fifteenth barrier wall 22-15, partition 20-16 formed by the sixteenth barrier wall 22-16, partition 20-17 formed by the seventeenth barrier wall 22-17, partition 20-18 formed by the eighteenth barrier wall 22-18, partition 20-19 formed by the nineteenth barrier wall 22-19, partition 20-20 formed by the twentieth barrier wall 22-20, partition 20-21 formed by the twenty first barrier wall 22-21, partition 20-22 formed by the twenty second barrier wall 22-22, partition 20-23 formed by the twenty third barrier wall 22-23, and partition 20-24 formed by the twenty fourth barrier wall 22-24, as partition 20. The partition 20 is arranged in the partitioned member 120 in a lattice pattern. Specifically, a plurality of partitions 20 are arranged along the direction of the long side of the partitioned member 120 and a plurality are arranged along the direction of the short side of the partitioned member 120. That is, the partition 20 is arranged in a lattice pattern along the X axis and the Y axis. FIG 1 illustrates a partitioned member 120 formed with a total of 24 partitions with six columns thereof along the direction of the long side of the partitioned member 120 and four rows thereof along the direction of the short side of the partitioned member 120.

Note that while FIG. 1 describes an example of a partitioned member having 24 barrier walls, that is, a partitioned member having 24 partitions, the number of barrier walls (number of partitions) the partitioned member 120 can have is not limited to this.

Note that while FIG. 1 describes an example of a partitioned member 120 formed in a lattice pattern, the arrangement of the barrier walls (arrangement of the partitions) in the partitioned member 120 is not limited to this.

The barrier wall 22 has a first surface 22a, a second surface 22b, a third surface 22c, and a fourth surface 22d.

The first surface 22a of the barrier wall 22 is a surface that is parallel to the XY plane. The first surface 22a of the barrier wall 22 is included in the first surface 120a of the partitioned member 120. That is, the first surface 22a of the barrier wall 22 is formed in the same position in the Z axis direction as the first surface 120a of the partitioned member 120. When the plate 100 is formed by joining the partitioned member 120 with the board 130, the first surface 22a of the barrier wall 22 is a surface that is substantially parallel to a second surface 130b of the board 130.

The second surface 22b of the barrier wall 22 is a surface that is parallel to the XY plane. The second surface 22b of the barrier wall 22 is formed in a different position in the Z axis direction than the first surface 22a of the barrier wall 22. The second surface 22b of the barrier wall 22 is parallel to the first surface 22a of the barrier wall 22. When the plate 100 is formed by joining the partitioned member 120 with the board 130, the first surface 22a of the barrier wall 22 is positioned more to the Z axis plus side than is the second surface 22b of the barrier wall 22. The second surface 22b of the barrier wall 22 is the surface used for joining with the board 130 by welding. A welded part f, which will be described later, includes the second surface 22b of the barrier wall 22.

The third surface 22c of the barrier wall 22 is a surface formed along the outer edge of the first surface 22a of the barrier wall 22 and the outer edge of the second surface 22b of the barrier wall 22. The third surface 22c of the barrier wall 22 is a surface that includes a surface that is parallel to the YZ plane and a surface that is parallel to the ZX plane. The third surface 22c of the barrier wall 22 is an inner wall surface of the barrier wall 22. The third surface 22c of the barrier wall 22 is a surface that regulates the partition 20.

The fourth surface 22d of the barrier wall 22 is a surface formed along the outer edge of the first surface 22a of the barrier wall 22 and the outer edge of the second surface 22b of the barrier wall 22. The fourth surface 22d of the barrier wall 22 is a surface that includes a surface that is parallel to the YZ plane and a surface that is parallel to the ZX plane. The fourth surface 22d of the barrier wall 22 is a surface that is on the opposite side from the third surface 22c of the barrier wall 22. The fourth surface 22d of the barrier wall 22 is an outer wall surface of the barrier wall 22.
The linking part 24 is a member that links the barrier wall 22. Specifically, the linking part 24 is a member that links the barriers walls from the first barrier wall 22-1 to the twenty fourth barrier wall 22-24.

Each of the barrier walls from the first barrier wall 22-1 to the twenty fourth barrier wall 22-24 is linked to the other barriers walls by the linking part 24.

Specifically, each of the barrier walls from the first barrier wall 22-1 to the twenty fourth barrier wall 22-24 is linked to a neighboring barrier wall by the linking part 24.

As an example, the first barrier wall 22-1 is linked to the second barrier wall 22-2 by the linking part 24. The first barrier wall 22-1 is linked to the fifth barrier wall 22-5 by the linking part 24. Therefore, the first barrier wall 22-1 is linked to two barrier walls - the second barrier wall 22-2 and the fifth barrier wall 22-5 - by the linking part 24.

As another example, the second barrier wall 22-2 is linked to the first barrier wall 22-1 by the linking part 24. The second barrier wall 22-2 is linked to the third barrier wall 22-3 by the linking part 24. The second barrier wall 22-2 is linked to the sixth barrier wall 22-6 by the linking part 24. Therefore, the second barrier wall 22-2 is linked to three barrier walls - the first barrier wall 22-1, the third barrier wall 22-3, and the sixth barrier wall 22-6 - by the linking part 24.

As another example, the sixth barrier wall 22-6 is linked to the second barrier wall 22-2 by the linking part 24. The sixth barrier wall 22-6 is linked to the fifth barrier wall 22-5 by the linking part 24. The sixth barrier wall 22-6 is linked to the seventh barrier wall 22-7 by the linking part 24. The sixth barrier wall 22-6 is linked to the tenth barrier wall 22-10 by the linking part 24. Therefore, the sixth barrier wall 22-6 is linked to four barrier walls - the second barrier wall 22-2, the fifth barrier wall 22-5, the seventh barrier wall 22-7, and the tenth barrier wall 22-10 - by the linking part 24.

More specifically, each of the barrier walls from the first barrier wall 22-1 to the twenty fourth barrier wall 22-24 is linked to a neighboring barrier wall and to barrier walls other than the neighboring barrier wall by the linking part 24.

As an example, the first barrier wall 22-1 is linked to all the barriers walls from the second barrier wall 22-2 to the twenty fourth barrier wall 22-24 by the linking part 24. In this way, the partitioned member 120 is integrated by the fact that the plurality of barrier walls are linked by the linking part 24, and thus the partition 20 is formed.

The linking part 24 has a first surface 24a and a second surface 24b.

The first surface 24a of the linking part 24 is a surface that is parallel to the XY plane. The first surface 24a of the linking part 24 is included in the first surface 120a of the partitioned member 120. That is, the first surface 24a of the linking part 24 is formed in the same position in the Z axis direction as the first surface 120a of the partitioned member 120. When the plate 100 is formed by joining the partitioned member 120 with the board 130, the first surface 24a of the linking part 24 is a surface that is substantially parallel to the second surface 130b of the board 130.

The second surface 24b of the linking part 24 is a surface that is parallel to the XY plane. The second surface 24b of the linking part 24 is formed in a different position in the Z axis direction than the first surface 24a of the linking part 24. The second surface 24b of the linking part 24 is parallel to the first surface 24a of the linking part 24. The second surface 24b of the linking part 24 is formed in a different position in the Z axis direction than the second surface 22b of the barrier wall 22. Specifically, the second surface 24b of the linking part 24 is formed between the first surface 22a of the barrier wall 22 and the second surface 22b of the barrier wall 22 in the Z axis direction.

The partition 20 is formed in the partitioned member 120 in a state forming a gap 28 between a barrier wall and another barrier wall.

This will be described taking the first barrier wall 22-1 as an example.

A fourth surface 22-1d of the first barrier wall 22-1 is separated from a fourth surface 22-2d of the second barrier wall 22-2. The fourth surface 22-1d of the first barrier wall 22-1 and the fourth surface 22-2d of the second barrier wall 22-2 are separated only by the amount interposed by the linking part 24. That is, the gap 28 includes the space formed by the second surface 24b of the linking part 24 between the fourth surface 22-1d of the first barrier wall 22-1 and the fourth surface 22-2d of the second barrier wall 22-2.

The fourth surface 22-1d of the first barrier wall 22-1 is separated from a fourth surface 22-5d of the fifth barrier wall 22-5. The fourth surface 22-1d of the first barrier wall 22-1 and the fourth surface 22-5d of the fifth barrier wall 22-5 are separated only by the amount interposed by the linking part 24. That is, the gap 28 includes the space formed by the second surface 24b of the linking part 24 between the fourth surface 22-1d of the first barrier wall 22-1 and the fourth surface 22-5d of the fifth barrier wall 22-5.

The fourth surface 22-1d of the first barrier wall 22-1 is separated from a fourth surface 22-6d of the sixth barrier wall 22-6. The fourth surface 22-1d of the first barrier wall 22-1 and the fourth surface 22-6d of the sixth barrier wall 22-6 are separated only by the amount interposed by the linking part 24. That is, the gap 28 includes the space formed by the second surface 24b of the linking part 24 between the fourth surface 22-1d of the first barrier wall 22-1 and the fourth surface 22-6d of the sixth barrier wall 22-6.

The first barrier wall 22-1 forms the partition 20-1 in a state where the gap 28 is formed between all of the linked barrier walls - the second barrier wall 22-2, the fifth barrier wall 22-5, and the sixth barrier wall 22-6.

This will be described taking the second barrier wall 22-2 as an example.

The fourth surface 22-2d of the second barrier wall 22-2 is separated from the fourth surface 22-1d of the first barrier wall 22-1. The fourth surface 22-2d of the second barrier wall 22-2 and the fourth surface 22-1d of the first barrier wall 22-1 are separated only by the amount interposed by the linking part 24. That is, the gap 28 includes the space formed by the second surface 24b of the linking part 24 between the fourth surface 22-2d of the second barrier wall 22-2 and the fourth surface 22-1d of the first barrier wall 22-1.

The fourth surface 22-2d of the second barrier wall 22-2 is separated from the fourth surface 22-3d of the third barrier wall 22-3. The fourth surface 22-2d of the second barrier wall 22-2 and the fourth surface 22-3d of the third barrier wall 22-3 are separated only by the amount interposed by the linking part 24. That is, the gap 28 includes the space formed by the second surface 24b of the linking part 24 between the fourth surface 22-2d of the second barrier wall 22-2 and the fourth surface 22-3d of the third barrier wall 22-3.

The fourth surface 22-2d of the second barrier wall 22-2 is separated from the fourth surface 22-5d of the fifth barrier wall 22-5. The fourth surface 22-2d of the second barrier wall 22-2 and the fourth surface 22-5d of the fifth barrier wall 22-5 are separated only by the amount interposed by the linking part 24. That is, the gap 28 includes the space formed by the second surface 24b of the linking part 24 between the fourth surface 22-2d of the second barrier wall 22-2 and the fourth surface 22-5d of the fifth barrier wall 22-5.

The fourth surface 22-2d of the second barrier wall 22-2 is separated from the fourth surface 22-6d of the sixth barrier wall 22-6. The fourth surface 22-2d of the second barrier wall 22-2 and the fourth surface 22-6d of the first barrier wall 22-6 are separated only by the amount interposed by the linking part 24. That is, the gap 28 includes the space formed by the second surface 24b of the linking part 24 between the fourth surface 22-2d of the second barrier wall 22-2 and the fourth surface 22-6d of the sixth barrier wall 22-6.

The fourth surface 22-2d of the second barrier wall 22-2 is separated from a fourth surface 22-7d of the seventh barrier wall 22-7. The fourth surface 22-2d of the second barrier wall 22-2 and the fourth surface 22-7d of the seventh barrier wall 22-7 are separated only by the amount interposed by the linking part 24. That is, the gap 28 includes the space formed by the second surface 24b of the linking part 24 between the fourth surface 22-2d of the second barrier wall 22-2 and the fourth surface 22-7d of the seventh barrier wall 22-7.

The second barrier wall 22-2 forms the partition 20-2 in a state where the gap 28 is formed between all of the linked barrier walls - the first barrier wall 22-1, the third barrier wall 22-3, the fifth barrier wall 22-5, and the sixth barrier wall 22-6.

This will be described taking the sixth barrier wall 22-6 as an example.

The fourth surface 22-6d of the sixth barrier wall 22-6 is separated from the fourth surface 22-1d of the first barrier wall 22-1. The fourth surface 22-6d of the sixth barrier wall 22-6 and the fourth surface 22-1d of the first barrier wall 22-1 are separated only by the amount interposed by the linking part 24. That is, the gap 28 includes the space formed by the second surface 24b of the linking part 24 between the fourth surface 22-6d of the sixth barrier wall 22-6 and the fourth surface 22-1d of the first barrier wall 22-1.

The fourth surface 22-6d of the sixth barrier wall 22-6 is separated from the fourth surface 22-2d of the second barrier wall 22-2. The fourth surface 22-6d of the sixth barrier wall 22-6 and the fourth surface 22-2d of the second barrier wall 22-2 are separated only by the amount interposed by the linking part 24. That is, the gap 28 includes the space formed by the second surface 24b of the linking part 24 between the fourth surface 22-6d of the sixth barrier wall 22-6 and the fourth surface 22-2d of the second barrier wall 22-2.

The fourth surface 22-6d of the sixth barrier wall 22-6 is separated from the fourth surface 22-3d of the third barrier wall 22-3. The fourth surface 22-6d of the sixth barrier wall 22-6 and the fourth surface 22-3d of the third barrier wall 22-3 are separated only by the amount interposed by the linking part 24. That is, the gap 28 includes the space formed by the second surface 24b of the linking part 24 between the fourth surface 22-6d of the sixth barrier wall 22-6 and the fourth surface 22-3d of the third barrier wall 22-3.

The fourth surface 22-6d of the sixth barrier wall 22-6 is separated from the fourth surface 22-5d of the fifth barrier wall 22-5. The fourth surface 22-6d of the sixth barrier wall 22-6 and the fourth surface 22-5d of the fifth barrier wall 22-5 are separated only by the amount interposed by the linking part 24. That is, the gap 28 includes the space formed by the second surface 24b of the linking part 24 between the fourth surface 22-6d of the sixth barrier wall 22-6 and the fourth surface 22-5d of the fifth barrier wall 22-5.

The fourth surface 22-6d of the sixth barrier wall 22-6 is separated from the fourth surface 22-7d of the seventh barrier wall 22-7. The fourth surface 22-6d of the sixth barrier wall 22-6 and the fourth surface 22-7d of the seventh barrier wall 22-7 are separated only by the amount interposed by the linking part 24. That is, the gap 28 includes the space formed by the second surface 24b of the linking part 24 between the fourth surface 22-6d of the sixth barrier wall 22-6 and the fourth surface 22-7d of the seventh barrier wall 22-7.

The fourth surface 22-6d of the sixth barrier wall 22-6 is separated from a fourth surface 22-9d of the ninth barrier wall 22-9. The fourth surface 22-6d of the sixth barrier wall 22-6 and the fourth surface 22-9d of the ninth barrier wall 22-9 are separated only by the amount interposed by the linking part 24. That is, the gap 28 includes the space formed by the second surface 24b of the linking part 24 between the fourth surface 22-6d of the sixth barrier wall 22-6 and the fourth surface 22-9d of the ninth barrier wall 22-9.

The fourth surface 22-6d of the sixth barrier wall 22-6 is separated from a fourth surface 22-10d of the tenth barrier wall 22-10. The fourth surface 22-6d of the sixth barrier wall 22-6 and the fourth surface 22-10d of the tenth barrier wall 22-10 are separated only by the amount interposed by the linking part 24. That is, the gap 28 includes the space formed by the second surface 24b of the linking part 24 between the fourth surface 22-6d of the sixth barrier wall 22-6 and the fourth surface 22-10d of the tenth barrier wall 22-10.

The fourth surface 22-6d of the sixth barrier wall 22-6 is separated from a fourth surface 22-11d of the eleventh barrier wall 22-11. The fourth surface 22-6d of the sixth barrier wall 22-6 and the fourth surface 22-11d of the eleventh barrier wall 22-11 are separated only by the amount interposed by the linking part 24. That is, the gap 28 includes the space formed by the second surface 24b of the linking part 24 between the fourth surface 22-6d of the sixth barrier wall 22-6 and the fourth surface 22-11d of the eleventh barrier wall 22-11.

The sixth barrier wall 22-6 forms the partition 20-6 in a state where a gap 28 is formed between all of the linked barrier walls - the first barrier wall 22-1, second barrier wall 22-2, the third barrier wall 22-3, the fifth barrier wall 22-5, the seventh barrier wall 22-7, the ninth barrier wall 22-9, the tenth barrier wall 22-10, and the eleventh barrier wall 22-11.

In this way the gap 28 is a space formed between a barrier wall and another barrier wall. The gap 28 is a space surrounded by the barrier wall 22 and the linking part 24.

The reinforcing part 26 is a member that reinforces the mechanical strength of the partitioned member 120. The reinforcing part 26 is a member that forms the outer edge of the partitioned member 120. The reinforcing part 26 is connected to the barrier wall, of the barrier wall that 22 configures the partitioned member 120, that is positioned on the outermost circumference. The reinforcing part 26 stretches across the outermost circumference of the barrier wall. Specifically, the reinforcing part 26 stretches across the entire circumference of the partitioned member 120. In FIG 1, the reinforcing part 26 is connected to the first barrier wall 22-1, the second barrier wall 22-2, the third barrier wall 22-3, the fourth barrier wall 22-4, the fifth barrier wall 22-5, the eighth barrier wall 22-8, the ninth barrier wall 22-9, the twelfth barrier wall 22-12, the thirteenth barrier wall 22-13, the sixteen barrier wall 22-16, the seventeenth barrier wall 22-17, the twentieth barrier wall 22-20, the twenty first barrier wall 22-21, the twenty second barrier wall 22-22, the twenty third barrier wall 22-23, and the twenty fourth barrier wall 22-24. Note that the reinforcing part 26 can be provided, for example, along one long or short side of the partitioned member 120, which has a rectangular geometry, rather than the entire circumference of the partitioned member 120. In this way, the mechanical strength of the outer edge portion of the partitioned member 120 is reinforced by the reinforcing part 26.

The reinforcing part 26 is a linking member that links the first barrier wall 22-1, the second barrier wall 22-2, the third barrier wall 22-3, the fourth barrier wall 22-4, the fifth barrier wall 22-5, the eighth barrier wall 22-8, the ninth barrier wall 22-9, the twelfth barrier wall 22-12, the thirteenth barrier wall 22-13, the sixteen barrier wall 22-16, the seventeenth barrier wall 22-17, the twentieth barrier wall 22-20, the twenty first barrier wall 22-21, the twenty second barrier wall 22-22, the twenty third barrier wall 22-23, and the twenty fourth barrier wall 22-24. The reinforcing part 26 functions as a linking part that links the barrier wall 22.

The reinforcing part 26 is also a gripping member in the partitioned member 120. The reinforcing part 26 functions as a gripping part that grips the partitioned member 120.

Note that the partitioned member 120 may be configured without the reinforcing part 26.

The partitioned member 120 is configured of, for example, a resin. Specifically, the partitioned member 120 is configured of a thermally reversible resin. The partitioned member 120 is, for example, a translucent material. Specifically, the partitioned member 120 is a transparent material. Examples of resins used in the partitioned member 120 include acrylic resins such as poly methyl methacrylate resins and the like, polycarbonate (PC), cycloolefin copolymers (COC), polystyrene (PS) and the like.

The board 130 is a plate member. The board 130 has a first surface 130a and a second surface 130b. The first surface 130a of the board 130 is a surface that is parallel to the XY plane. In order to ensure a desired flatness, the first surface 130a of the board 130 is polished to an accuracy of, for example, several microns.

The second surface 130b of the board 130 is a surface that is parallel to the XY plane. The second surface 130b of the board 130 is formed in a different position in the Z axis direction than the first surface 130a of the board 130. The second surface 130b of the board 130 is parallel to the first surface 130a of the board 130. When the board 130 is joined with the partitioned member 120, the first surface 130a of the board 130 is positioned more to the Z plus side than is the second surface 130b of the board 130. In order to ensure a desired flatness, the second surface 130b of the board 130 is polished to an accuracy of, for example, several microns. When the plate 100 is formed by joining the partitioned member 120 with the board 130, the second surface 130b of the board 130 is a surface that is substantially parallel to the first surface 120a of the partitioned member 120.

The board 130 is configured of, for example, a resin. Specifically, the board 130 is configured of a thermally reversible resin. The board 130 is, for example, a translucent material. Specifically, the board 130 is a transparent material. Examples of resins used in the board 130 include acrylic resins such as poly methyl methacrylate resins and the like, polycarbonate (PC), cycloolefin copolymers (COC), and polystyrene (PS).

The board 130 is joined with the partitioned member 120. Specifically, the first surface 130a of the board 130 is joined with the second surface 120b of the partitioned member 120. More specifically, the first surface 130a of the board 130 is joined with the second surface 120b of the partitioned member 120 so as to entirely cover the second surface 120b of the partitioned member 120. The second surface 120b of the partitioned member 120 and the first surface 130a of the board 130 are joined in a state where the partition 20 configured on the partitioned member 120 is closed from the second surface 120b side of the partitioned member 120 by the first surface 130a of the board 130.

A welded joint can exemplify the method for joining the partitioned member 120 and the board 130. In this case, the partitioned member 120 and the board 130 are configured of thermally reversible resin. The partitioned member 120 and the board 130 are joined together by heating a joining location of the partitioned member 120 and a joining location of the board 130 to the melting point and then applying pressure. The elution of undesirable chemical compounds can be suppressed better when the partitioned member 120 and the board 130 are joined by welding than when the partitioned member 120 and the board 130 are joined using an adhesive. The stability of the joint can be improved more when the partitioned member 120 and the board 130 are joined by welding than when the partitioned member 120 and the board 130 are joined by an adhesive. The partitioned member 120 is welded to the board 130 by the entire second surface 120b of the partitioned member 120. That is, the second surface 120b of the partitioned member 120 is welded to the first surface 130a of the board 130 across the entire second surface 22b of the barrier wall 22 that configures a well 110. Welding using the entire surface of the second surface 22b of the barrier wall 22 forms a sealing structure in the plate 100 such that a fluid sample stored in the well 110 will not leak from the well 110.

For example, ultrasonic welding can be applied to the welded joint between the partitioned member 120 and the board 130. Note that laser welding, vibration welding and the like may also be applied to the welded joint between the partitioned member 120 and the board 130.

When the partitioned member 120 and the board 130 are joined by welding, it is preferable that the material used in the partitioned member 120 have the same melting point as the melting point of the material used in the board 130. Therefore, it is preferable that the material used in the partitioned member 120 be the same as the material used in the board 130.

As an example, a poly methyl methacrylate resin can be used as the material in both the partitioned member 120 and the board 130.

As another example, polycarbonate (PC) can be used as the material in both the partitioned member 120 and the board 130.

As another example, a cycloolefin copolymer (COC) can be used as the material in both the partitioned member 120 and the board 130.

As another example, polystyrene (PS) can be used as the material in both the partitioned member 120 and the board 130.

When the partitioned member 120 and the board 130 are joined by welding, it is preferable that the material used in the partitioned member 120 have a melting point that is close to the melting point of the material used in the board 130.

As an example, a poly methyl methacrylate resin can be used as the material for the partitioned member 120 and polycarbonate (PC) can be used as the material for the board 130.

As another example, polycarbonate (PC) can be used as the material for the partitioned member 120 and a poly methyl methacrylate resin can be used as the material for the board 130.

The plate 100 has the well 110 that was formed by the barrier wall 22 of the partitioned member 120 and the first surface 130a of the board 130. The first surface 130a of the board 130 configures the bottom of the well 110. The well is able to store, for example, a liquid sample and the like.

The plate 100 includes first well 110-1, second well 110-2, third well 110-3, fourth well 110-4, fifth well 110-5, sixth well 110-6, seventh well 110-7, eighth well 110-8, ninth well 110-9, tenth well 110-10, eleventh well 110-11, twelfth well 110-12, thirteenth well 110-13, fourteenth well 110-14, fifteenth well 110-15, sixteenth well 110-16, seventeenth well 110-17, eighteenth well 110-18, nineteenth well 110-19, twentieth well 110-20, twenty first well 110-21, twenty second well 110-22, twenty third well 110-23, and twenty fourth well 110-24.

The first well 110-1 is formed by the first barrier wall 22-1 and the board 130. Specifically, the first well 110-1 is formed by the third surface 22-1c of the first barrier wall 22-1 and the first surface 130a of the board 130.

The second well 110-2 is formed by the second barrier wall 22-2 and the board 130. Specifically, the second well 110-2 is formed by the third surface 22-2c of the second barrier wall 22-2 and the first surface 130a of the board 130.

The third well 110-3 is formed by the third barrier wall 22-3 and the board 130. Specifically, the third well 110-3 is formed by the third surface 22-3c of the third barrier wall 22-3 and the first surface 130a of the board 130.

The fourth well 110-4 is formed by the fourth barrier wall 22-4 and the board 130. Specifically, the fourth well 110-4 is formed by the third surface 22-4c of the fourth barrier wall 22-4 and the first surface 130a of the board 130.

The fifth well 110-5 is formed by the fifth barrier wall 22-5 and the board 130. Specifically, the fifth well 110-5 is formed by the third surface 22-5c of the fifth barrier wall 22-5 and the first surface 130a of the board 130.

The sixth well 110-6 is formed by the sixth barrier wall 22-6 and the board 130. Specifically, the sixth well 110-6 is formed by the third surface 22-6c of the sixth barrier wall 22-6 and the first surface 130a of the board 130.

The seventh well 110-7 is formed by the seventh barrier wall 22-7 and the board 130. Specifically, the seventh well 110-7 is formed by the third surface 22-7c of the seventh barrier wall 22-7 and the first surface 130a of the board 130.

The eighth well 110-8 is formed by the eighth barrier wall 22-8 and the board 130. Specifically, the eighth well 110-8 is formed by the third surface 22-8c of the eighth barrier wall 22-8 and the first surface 130a of the board 130.

The ninth well 110-9 is formed by the ninth barrier wall 22-9 and the board 130. Specifically, the ninth well 110-9 is formed by the third surface 22-9c of the ninth barrier wall 22-9 and the first surface 130a of the board 130.

The tenth well 110-10 is formed by the tenth barrier wall 22-10 and the board 130. Specifically, the tenth well 110-10 is formed by the third surface 22-10c of the tenth barrier wall 22-10 and the first surface 130a of the board 130.

The eleventh well 110-11 is formed by the eleventh barrier wall 22-11 and the board 130. Specifically, the eleventh well 110-11 is formed by the third surface 22-11c of the eleventh barrier wall 22-11 and the first surface 130a of the board 130.

The twelfth well 110-12 is formed by the twelfth barrier wall 22-12 and the board 130. Specifically, the twelfth well 110-12 is formed by the third surface 22-12c of the twelfth barrier wall 22-12 and the first surface 130a of the board 130.

The thirteenth well 110-13 is formed by the thirteenth barrier wall 22-13 and the board 130. Specifically, the thirteenth well 110-13 is formed by the third surface 22-13c of the thirteenth barrier wall 22-13 and the first surface 130a of the board 130.

The fourteenth well 110-11 is formed by the fourteenth barrier wall 22-14 and the board 130. Specifically, the fourteenth well 110-14 is formed by the third surface 22-14c of the fourteenth barrier wall 22-14 and the first surface 130a of the board 130.

The fifteenth well 110-15 is formed by the fifteenth barrier wall 22-15 and the board 130. Specifically, the fifteenth well 110-15 is formed by the third surface 22-15c of the fifteenth barrier wall 22-15 and the first surface 130a of the board 130.

The sixteenth well 110-16 is formed by the sixteenth barrier wall 22-16 and the board 130. Specifically, the sixteenth well 110-16 is formed by the third surface 22-16c of the sixteenth barrier wall 22-16 and the first surface 130a of the board 130.

The seventeenth well 110-17 is formed by the seventeenth barrier wall 22-17 and the board 130. Specifically, the seventeenth well 110-17 is formed by the third surface 22-17c of the seventeenth barrier wall 22-17 and the first surface 130a of the board 130.

The eighteenth well 110-18 is formed by the eighteenth barrier wall 22-18 and the board 130. Specifically, the eighteenth well 110-18 is formed by the third surface 22-18c of the eighteenth barrier wall 22-18 and the first surface 130a of the board 130.

The nineteenth well 110-19 is formed by the nineteenth barrier wall 22-19 and the board 130. Specifically, nineteenth well 110-19 is formed by the third surface 22-19c of the nineteenth barrier wall 22-19 and the first surface 130a of the board 130.

The twentieth well 110-20 is formed by the twentieth barrier wall 22-20 and the board 130. Specifically, the twentieth well 110-20 is formed by the third surface 22-20c of the twentieth barrier wall 22-20 and the first surface 130a of the board 130.

The twenty first well 110-21 is formed by the twenty first barrier wall 22-21 and the board 130. Specifically, the twenty first well 110-21 is formed by the third surface 22-21c of the twenty first barrier wall 22-21 and the first surface 130a of the board 130.

The twenty second well 110-22 is formed by the twenty second barrier wall 22-2 and the board 130. Specifically, the twenty second well 110-22 is formed by the third surface 22-22c of the twenty second barrier wall 22-22 and the first surface 130a of the board 130.

The twenty third well 110-23 is formed by the twenty third barrier wall 22-23 and the board 130. Specifically, the twenty third well 110-23 is formed by the third surface 22-23c of the twenty third barrier wall 22-23 and the first surface 130a of the board 130.

The twenty fourth well 110-24 is formed by the twenty fourth barrier wall 22-24 and the board 130. Specifically, the twenty fourth well 110-24 is formed by the third surface 22-24c of the twenty fourth barrier wall 22-24 and the first surface 130a of the board 130.

FIG 2 is a bottom surface view of the plate 100. When the board 130 is configured of a translucent member, a welded part 35 created by welding the partitioned member 120 and the board 130 together can be observed from the second surface 130b (the bottom surface of the plate 100) of the board 130. The welded part 35 occurs at the joint portion where the partitioned member 120 and the board 130 were welded together and joined. Specifically, because the partitioned member 120 and the board 130 are welded together across the entire surface of the second surface 22b of the barrier wall 22, the welded part 35 emerges with substantially the same shape as the second surface 22b of the barrier wall 22. That is, a welded part 35 of the same shape, quantity and arrangement as the second surface 22b of the barrier wall 22 can be confirmed from the second surface 130b (the bottom surface of the plate 100) of the board 130. The shape of the welded part 35 emerges in a shape that corresponds with the shape of the welded part 22f. Incidentally, the welded part 35 is sometimes referred to as a welded layer.

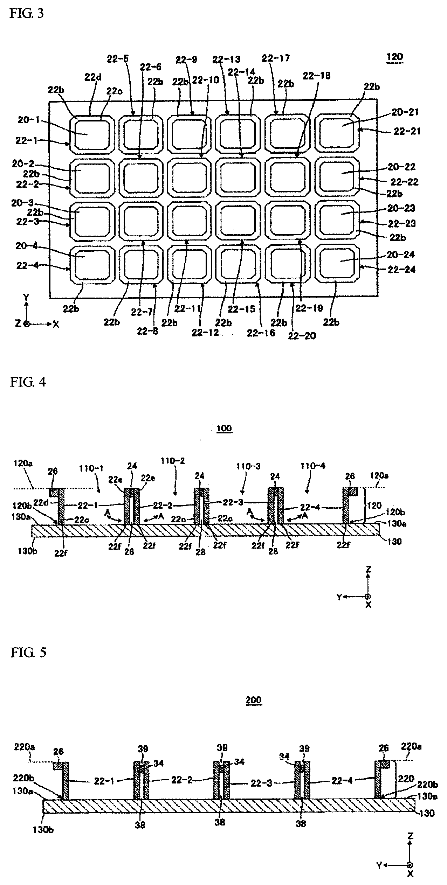

FIG 3 is a bottom surface view of the partitioned member 120. The partitioned member 120 has the barrier wall 22. The barrier wall 22 forms a partition on the inside thereof that is a through hole. The barrier wall 22 is arranged in a lattice pattern along the X axis and the Y axis. The first barrier wall 22-1, the second barrier wall 22-2, the third barrier wall 22-3, the fourth barrier wall 22-4, the fifth barrier wall 22-5, the sixth barrier wall 22-6, the seventh barrier wall 22-7, the eighth barrier wall 22-8, the ninth barrier wall 22-9, the tenth barrier wall 22-10, the eleventh barrier wall 22-11, the twelfth barrier wall 22-12, the thirteenth barrier wall 22-13, the fourteenth barrier wall 22-14, the fifteenth barrier wall 22-15, the sixteenth barrier wall 22-16, the seventeenth barrier wall 22-17, the eighteenth barrier wall 22-18, the nineteenth barrier wall 22-19, the twentieth barrier wall 22-20, the twenty first barrier wall 22-21, the twenty second barrier wall 22-22, the twenty third barrier wall 22-23, and the twenty fourth barrier wall 22-24 are illustrated in FIG. 3 arranged along the long side of the partitioned member 120 in six columns and along the short side of the partitioned member 120 in four rows. Each of the barrier walls, from the first barrier wall 22-1 to the twenty fourth 22-24, is linked to the other barrier walls by the linking part 24. That is, in FIG 3, the first barrier wall 22-1 through the twenty fourth barrier wall 22-24 are coupled in one body by the linking part 24. The outer edge portion of the partitioned member 120 is reinforced by the reinforcing part 26.

The partitioned member 120 has the welded part 22f for welding the partitioned member 120 to the board 130. The welded part 22f is provided on the end of the barrier wall 22. The welded part 22f is the tip portion on the second surface 22b side of the barrier wall 22 that includes the second surface 22b of the barrier wall 22. The welded part 22f is the tip portion on the lower edge side, that is, the Z minus direction side of the barrier wall 22. The welded part 22b extends along the entire circumference of this tip portion. For example, in FIG 3, each of the barrier walls from the first barrier wall 22-1 to the twenty fourth barrier wall 22-24 has a shape that is a substantially square shape and the welded part 22f extends along the entire outer circumference of the square. The partitioned member 120 is welded to the first surface 130a of the board 130 across the entire outer circumference of the tip portion on the second surface 22b side of the barrier wall 22. Incidentally, the welded part 22f is sometimes referred to as a welded layer.

FIG 4 is a cross sectional view of the plate 100. FIG. 4 illustrates a cross section of the plate 100 cut along the cut line A-A in FIG 1. The plate 100 has the well 110 that was formed by the barrier wall 22 of the partitioned member 120 and the board 130. Four wells - the first well 110-1 formed by the first barrier wall 22-1 of the partitioned member 120 and the first surface 130a of the board 130, the second well 110-2 formed by the second barrier wall 22-2 of the partitioned member 120 and the first surface 130a of the board 130, the third well 110-3 formed by the third barrier wall 22-3 of the partitioned member 120 and the first surface 130a of the board 130, and the fourth well 110-4 formed by the fourth barrier wall 22-4 of the partitioned member 120 and the first surface 130a of the board 130 - are illustrated in FIG 4. The barrier wall 22 is linked by the linking part 24. In FIG 4, the linking part 24 is provided between the first barrier wall 22-1 and the second barrier wall 22-2, between the second barrier wall 22-2 and the third barrier wall 22-3, and between the third barrier wall 22-3 and the fourth barrier wall 22-4. Specifically, the barrier wall 22 is linked by the linking part 24 in an upper end 22e on the Z plus direction side of the barrier wall 22, that is, on the first surface 120a side of the partitioned member 120. The barrier wall 22 is separated below (Z minus direction side) the linking part 24. The partitioned member 120 has the gap 28 below (Z minus direction side) the linking part 24. In the partitioned member 120, the gap 28 is a space surrounded by the barrier wall 22 and the linking part 24. In the plate 100, the gap 28 is a space surrounded by the barrier wall 22, the linking part 24, and the board 130.

The degree of freedom for the relative position of the well 110 on the second surface 120b side of the partitioned member 120 is higher when the gap 28 is present than when the gap 28 is not present. That is, as illustrated in FIG. 4, the partitioned member 120 has a degree of freedom to displace two adjacent wells (for example, the first well 110-1 and the second well 110-2) away from or closer to one another in the directions of the arrows A with the linking part 24 provided between adjacent barrier walls (for example, the first barrier wall 22-1 and the second barrier wall 22-2) as a fulcrum.

The rigidity of the partitioned member 120 is lower when the gap 28 is formed than when the gap 28 is not formed. Therefore, even if the partitioned member 120 is pressed against the board 130 when the partitioned member 120 and the board are joined, the pressing force from the partitioned member 120 does not readily transfer to the board 130 because the partitioned member 120 itself deforms. As a result, deformation (for example, deflection or distortion) can be suppressed from occurring in the board 130. Accordingly, a state where the board 130 does not deform can be maintained even after the partitioned member 120 and the board have been joined. By this, with the plate 100, it becomes possible to execute optical measurement of a sample in the well 110 with good accuracy.

The rigidity of the partitioned member 120 changes based on, for example, the depth (distance in the Z direction) and width (distance in the Y direction) of the gap 28. For example, because the linking part 24 is formed thinner as the gap 28 becomes deeper, the rigidity of the partitioned member 120 drops. Because the separation of the barrier wall 22 get larger as the width of the gap 28 gets wider, the rigidity of the partitioned member 120 drops.

In this way, because the plate is configured using a partitioned member that forms a partition in a state where a gap is formed between a barrier wall and another barrier wall, the surface accuracy of the plate can be kept from dropping.

When optical measurement of a sample is performed on the plate, the optical measurement can be performed with good accuracy.

Next, modified embodiments of the partitioned member 120 will be described with reference to FIG. 5 through FIG. 12.

FIG. 5 is a cross sectional view of a plate 200 that applies a partitioned member 220, which is a first modified embodiment of the partitioned member 120. Note that the same reference numerals are attached to configuring elements provided by the plate 200 that applies the partitioned member 220 of the first modified embodiment as were attached to configuring elements having the same function and configuration that were provided by the plate 100 that applied the partitioned member 120 described in conjunction with FIG 1 through FIG. 4. Descriptions of configuring elements having the same reference numerals as configuring elements of the partitioned member 120 described in conjunction with FIG 1 through FIG. 4 are sometimes omitted in the description of the plate 200 that applies the partitioned member 220 of the first modified embodiment so as to avoid redundant descriptions.

The plate 200 provides the partitioned member 220 and the board 130.

The partitioned member 220 provides the barrier wall 22, a linking part 34 and the reinforcing part 26. The partitioned member 220 has a first surface 220a and a second surface 220b. The first surface 220a of the partitioned member 220 is a surface that is parallel to the XY plane. The second surface 220b of the partitioned member 220 is a surface that is parallel to the XY plane. The first surface 220a of the partitioned member 220 is formed in a different position in the Z axis direction than the second surface 220b of the partitioned member 220. The first surface 220a of the partitioned member 220 is parallel to the second surface 220b of the partitioned member 220.

The linking part 34 is a member that links the barrier wall 22. The linking part 34 has a first surface 34a and a second surface 34b.

The first surface 34a of the linking part 34 is a surface that is parallel to the XY plane. The first surface 34a of the linking part 34 is formed in a different position in the Z axis direction than the first surface 220a of the partitioned member 220. Specifically, the first surface 34a of the linking part 34 is formed in a position more to the Z axis minus direction side than is the first surface 220a of the partitioned member 220.

The second surface 34b of the linking part 34 is a surface that is parallel to the XY plane. The second surface 34b of the linking part 34 is formed in a different position in the Z axis direction than the first surface 34a of the linking part 34. The second surface 34b of the linking part 34 is parallel to the first surface 34a of the linking part 34. The second surface 34b of the linking part 34 is formed in a different position in the Z axis direction than the second surface 22b of the barrier wall 22. Specifically, the second surface 34b of the linking part 34 is positioned between the first surface 34a of the linking part 34 and the second surface 22b of the barrier wall 22 in the Z axis direction.
The partitioned member 220 has a gap 38 formed between the barrier wall 22 and the first surface 34a of the lining part 34, and a gap 39 formed between the barrier wall 22 and the second surface 34b of the barrier wall 22. By forming the gap 39, the partitioned member 220 is able to suppress rigidity.

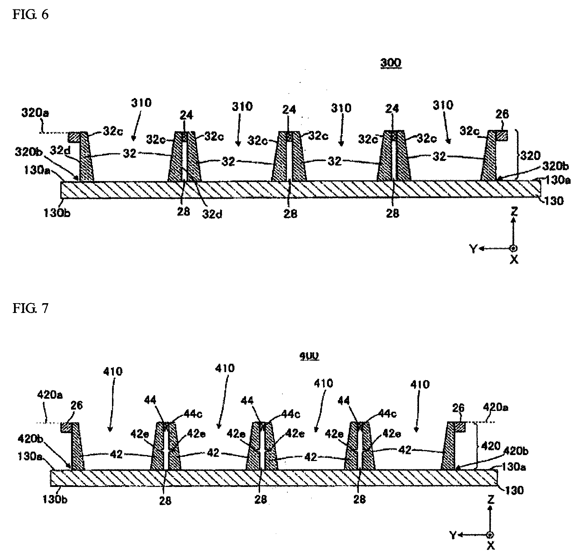

FIG 6 is a cross sectional view of a plate 300 that applies a partitioned member 320, which is a second modified embodiment of the partitioned member 120. Note that the same reference numerals are attached to configuring elements provided by the plate 300 that applies the partitioned member 320 of the second modified embodiment as were attached to configuring elements having the same function and configuration that were provided by the plate 100 that applied the partitioned member 120 described in conjunction with FIG 1 through FIG. 4. Descriptions of configuring elements having the same reference numerals as configuring elements of the partitioned member 120 described in conjunction with FIG 1 through FIG 4 are sometimes omitted in the description of the plate 300 that applies the partitioned member 320 of the second modified embodiment so as to avoid redundant descriptions.

The plate 300 provides the partitioned member 320 and the board 130.

The partitioned member 320 provides a barrier wall 32, the linking part 24 and the reinforcing part 26. The partitioned member 320 has a first surface 320a and a second surface 320b. The first surface 320a of the partitioned member 320 is a surface that is parallel to the XY plane. The second surface 320b of the partitioned member 320 is a surface that is parallel to the XY plane. The first surface 320a of the partitioned member 320 is formed in a different position in the Z axis direction than the second surface 320b of the partitioned member 320. The first surface 320a of the partitioned member 320 is parallel to the second surface 320b of the partitioned member 320.

The barrier wall 32 is a member that forms a partition 30. The partitioned member 320 has the partition 30, which is formed by the barrier wall 32.

The barrier wall 32 has a first surface 32a, a second surface 32b, a third surface 32c, and a fourth surface 32d.

The second surface 32a of the barrier wall 32 is a surface that is parallel to the XY plane. The first surface 32a of the barrier wall 32 includes the first surface 320a of the partitioned member 320. That is, the first surface 32a of the barrier wall 32 is formed in the same position in the Z axis direction as the first surface 320a of the partitioned member 320. When the plate 300 is formed by joining the partitioned member 320 with the board 130, the first surface 32a of the barrier wall 32 is a surface that is substantially parallel to the second surface 130b of the board 130.

The second surface 32b of the barrier wall 32 is a surface that is parallel to the XY plane. The second surface 32b of the barrier wall 32 is formed in a different position in the Z axis direction than the first surface 32a of the barrier wall 32. The second surface 32b of the barrier wall 32 is parallel to the first surface 32a of the barrier wall 32. When the plate 300 is formed by joining the partitioned member 320 with the board 130, the first surface 32a of the barrier wall 32 is positioned more to the Z axis plus side than is the second surface 32b of the barrier wall 32. The second surface 32b of the barrier wall 32 is the surface used for joining with the board 130 by welding.

The third surface 32c of the barrier wall 32 is a surface formed along the outer edge of the first surface 32a of the barrier wall 32 and the outer edge of the second surface 32b of the barrier wall 32. The third surface 32c of the barrier wall 32 is an inner wall surface of the barrier wall 32. The third surface 32c of the barrier wall 32 is a surface that regulates the partition 30.

The fourth surface 32d of the barrier wall 32 is a surface formed along the outer edge of the first surface 32a of the barrier wall 32 and the outer edge of the second surface 32b of the barrier wall 32. The fourth surface 32d of the barrier wall 32 is a surface that includes a surface that is parallel to the YZ plane and a surface that is parallel to the ZX plane. The fourth surface 32d of the barrier wall 32 is a surface that is on the opposite side from the third surface 32c of the barrier wall 32. The fourth surface 32d of the barrier wall 32 is an outer wall surface of the barrier wall 32.

As the third surface 32c of the barrier wall 32 approaches the second surface 32b of the barrier wall 32 from the first surface 32a of the barrier wall 32, the distance to the fourth surface 32d of the barrier wall 32 gets longer. The third surface 32c of the barrier wall 32 is slanted in relation to the fourth surface 32b of the barrier wall 32. The third surface 32c of the barrier wall 32 is not parallel to either the YZ plane or the ZX plane. The thickness of the barrier wall formed by the third surface 32c of the barrier wall 32 and the fourth surface 32d of the barrier wall 32 becomes thicker going from the first surface 32a of the barrier wall 32 toward the second surface 32b of the barrier wall 32.

The inner diameter of the partition 30 becomes shorter going from the first surface 320a of the partitioned member 320 toward the second surface 320b of the partitioned member 320.

The plate 300 has a well 310 that was formed by the barrier wall 32 of the partitioned member 320 and the first surface 130a of the board 130. The inner diameter of the well 310 gets shorter going toward a well bottom 310a.

This type of shape for the well 310 allows the amount of a specimen dispensed in the well 310 to be reduced.

Note that instead of a flat slanted surface, the third surface 32c of the barrier wall 32 may be a curved slanted surface.

FIG. 7 is a cross sectional view of a plate 400 that applies a partitioned member 420, which is a third modified embodiment of the partitioned member 120. Note that the same reference numerals are attached to configuring elements provided by the plate 400 that applies the partitioned member 420 of the third modified embodiment as were attached to configuring elements having the same function and configuration that were provided by the plate 100 that applied the partitioned member 120 described in conjunction with FIG. 1 through FIG. 4. Descriptions of configuring elements having the same reference numerals as configuring elements of the partitioned member 120 described in conjunction with FIG 1 through FIG 4 are sometimes omitted in the description of the plate 400 that applies the partitioned member 420 of the third modified embodiment so as to avoid redundant descriptions.

The plate 400 provides the partitioned member 420 and the board 130.

The partitioned member 420 provides a barrier wall 42, a linking part 44 and the reinforcing part 26. The partitioned member 420 has a first surface 420a and a second surface 420b. The first surface 420a of the partitioned member 420 is a surface that is parallel to the XY plane. The second surface 420b of the partitioned member 420 is a surface that is parallel to the XY plane. The first surface 420a of the partitioned member 420 is formed in a different position in the Z axis direction than the second surface 420b of the partitioned member 420. The first surface 420a of the partitioned member 420 is parallel to the second surface 420b of the partitioned member 420.

The barrier wall 42 is a member that forms a partition 40. The partitioned member 420 has the partition 40, which is formed by the barrier wall 42.

The barrier wall 42 has a first surface 42a, a second surface 42b, a third surface 42c, and a fourth surface 42d.

The first surface 42a of the barrier wall 42 is a surface that is parallel to the XY plane. The first surface 42a of the barrier wall 42 includes the first surface 420a of the partitioned member 420. That is, the first surface 42a of the barrier wall 42 is formed in the same position in the Z axis direction as the first surface 420a of the partitioned member 420. When the plate 400 is formed by joining the partitioned member 420 with the board 130, the first surface 42a of the barrier wall 42 is a surface that is substantially parallel to the second surface 130b of the board 130.

The second surface 42b of the barrier wall 42 is a surface that is parallel to the XY plane. The second surface 42b of the barrier wall 42 is formed in a different position in the Z axis direction than the first surface 42a of the barrier wall 42. The second surface 42b of the barrier wall 42 is parallel to the first surface 42a of the barrier wall 42. When the plate 300 is formed by joining the partitioned member 420 with the board 130, the first surface 42a of the barrier wall 42 is positioned more to the Z axis plus side than is the second surface 42b of the barrier wall 42. The second surface 42b of the barrier wall 42 is the surface used for joining with the board 130 by welding.

The third surface 42c of the barrier wall 42 is a surface formed along the outer edge of the first wall 42a of the barrier wall 42 and the outer edge of the second surface 42b of the barrier wall 42. The third surface 42c of the barrier wall 42 is an inner wall surface of the barrier wall 42. The third surface 42c of the barrier wall 42 is a surface that regulates the partition 40.

The fourth surface 42d of the barrier wall 42 is a surface formed along the outer edge of the first wall 42a of the barrier wall 42 and the outer edge of the second surface 42b of the barrier wall 42. The fourth surface 42d of the barrier wall 42 is a surface that includes a surface that is parallel to the YZ plane and a surface that is parallel to the ZX plane. The fourth surface 42d of the barrier wall 42 is a surface that is on the opposite side from the third surface 42c of the barrier wall 42. The fourth surface 42d of the barrier wall 42 is an outer wall surface of the barrier wall 42.

As the third surface 42c of the barrier wall 42 approaches the second surface 42b of the barrier wall 42 from the first surface 42a of the barrier wall 42, the distance to the fourth surface 42d of the barrier wall 42 gets longer. The third surface 42c of the barrier wall 42 is slanted in relation to the fourth surface 42b of the barrier wall 42. The third surface 42c of the barrier wall 42 is not parallel to either the YZ plane or the ZX plane. The thickness of the barrier wall formed by the third surface 42c of the barrier wall 42 and the fourth surface 42d of the barrier wall 42 becomes thicker going from the first surface 42a of the barrier wall 42 toward the second surface 42b of the barrier wall 42.

The inner diameter of the partition 40 becomes shorter going from the first surface 420a of the partitioned member 420 toward the second surface 420b of the partitioned member 420.

The barrier wall 42 has a cut 42e. Specifically, the barrier wall 42 has a cut in the fourth surface 42d of the barrier wall 42. The cut 42e includes a cut formed in the fourth surface 42d of the barrier wall 42 along the X direction, and a cut formed in the fourth surface 42d of the barrier wall 42 along the Y direction. The cut 42e is formed by providing a cut in, for example, the fourth surface 42d of the barrier wall 42. The width of the cut 42e is narrower than the width of the gap 28. The cut 42e functions a part of the gap 28.

The linking part 44 is a member that links the barrier wall 42.

The linking part 44 has a first surface 44a and a second surface 44b.

The first surface 44a of the linking part 44 is a surface that is parallel to the XY plane. The first surface 44a of the linking part 44 is included in the first surface 420a of the partitioned member 420. That is, the first surface 44a of the linking part 44 is formed in the same position in the Z axis direction as the first surface 420a of the partitioned member 420. When the plate 400 is formed by joining the partitioned member 420 with the board 130, the first surface 44a of the linking part 44 is a surface that is substantially parallel to the second surface 130b of the board 130.

The second surface 44b of the linking part 44 is a surface that is parallel to the XY plane. The second surface 42b of the linking part 44 is formed in a different position in the Z axis direction than the first surface 44a of the linking part 44. The second surface 44b of the linking part 44 is parallel to the first surface 44a of the linking part 44. The second surface 44b of the linking part 44 is formed in a different position in the Z axis direction than the second surface 42b of the barrier wall 42. Specifically, the second surface 44b of the linking part 44 is formed between the first surface 42a of the barrier wall 42 and the second surface 42b of the barrier wall 42 in the Z axis direction.

The linking part 44 has a cut 44c. Specifically, the linking part 44 has the cut 44c in the second surface 44b of the linking part 44. The cut 44c includes a cut formed in the second surface 44b of the linking part 44 along the X direction, and a cut formed in the second surface 44b of the linking part 44 along the Y direction. The cut 44c is formed by providing a cut in, for example, the fourth surface 44d of the linking part 44. The width of the cut 44c is narrower than the width of the gap 28. The cut 44c functions a part of the gap 28.

The plate 400 has a well 410 that was formed by the barrier wall 42 of the partitioned member 420 and the first surface 130a of the board 130. The inner diameter of the well 410 gets shorter going toward a well bottom 410a.

This type of shape for the well 410 allows the amount of a specimen dispensed in the well 410 to be reduced.

Note that instead of a flat slanted surface, the third surface 42c of the barrier wall 42 may be a curved slanted surface.

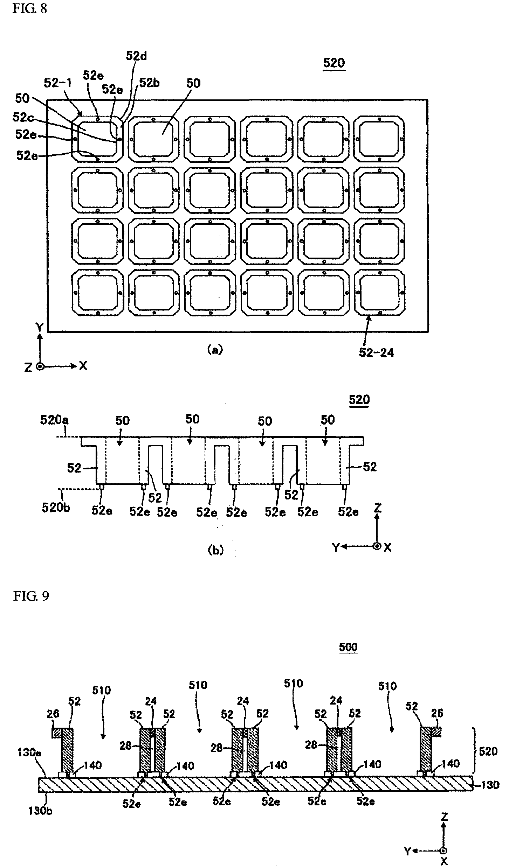

FIG 8 is a drawing illustrating a partitioned member 520, which is a fourth modified embodiment of the partitioned member 120. FIG 8 (a) is a bottom surface view of the partitioned member 520. FIG. 8 (b) is a side view of the partitioned member 520. Note that the same reference numerals are attached to configuring elements provided by the partitioned member 520 of the fourth modified embodiment as were attached to configuring elements having the same function and configuration that were provided by the partitioned member 120 described in conjunction with FIG. 1 through FIG 4. Descriptions of configuring elements having the same reference numerals as configuring elements of the partitioned member 120 described in conjunction with FIG 1 through FIG. 4 are sometimes omitted in the description of the partitioned member 520 of the fourth modified embodiment so as to avoid redundant descriptions.

The partitioned member 520 provides a barrier wall 52, the linking part 24 and the reinforcing part 26. The partitioned member 520 has a first surface 520a and a second surface 520b. The first surface 520a of the partitioned member 520 is a surface that is parallel to the XY plane. The second surface 520b of the partitioned member 520 is a surface that is parallel to the XY plane. The first surface 520a of the partitioned member 520 is formed in a different position in the Z axis direction than the second surface 520b of the partitioned member 520. The first surface 520a of the partitioned member 520 is parallel to the second surface 520b of the partitioned member 520.

The barrier wall 52 is a member that forms a partition 50. The partitioned member 520 has the partition 50, which is formed by the barrier wall 52.

The partitioned member 520 includes a total of 24 barrier walls, from the first barrier wall 52-1 to the twenty fourth barrier wall 52-24, as the barrier wall 52. The partitioned member 520 has a total of 24 partitions, formed by the first barrier wall 52-1 through the twenty fourth barrier wall 52-24, respectively, as the partition 50.

The barrier wall 52 has a first surface 52a, a second surface 52b, a third surface 52c, and a fourth surface 52d.

The first surface 52a of the barrier wall 52 is a surface that is parallel to the XY plane. The first surface 52a of the barrier wall 52 includes the first surface 520a of the partitioned member 520. That is, the first surface 52a of the barrier wall 52 is formed in the same position in the Z axis direction as the first surface 520a of the partitioned member 520. When the plate 500 is formed by joining the partitioned member 520 with the board 130, the first surface 52a of the barrier wall 52 is a surface that is substantially parallel to the second surface 130b of the board 130.

The second surface 52b of the barrier wall 52 is a surface that is parallel to the XY plane. The second surface 52b of the barrier wall 52 is formed in a different position in the Z axis direction than the first surface 52a of the barrier wall 52. The second surface 52b of the barrier wall 52 is parallel to the first surface 52a of the barrier wall 52. When the plate 500 is formed by joining the partitioned member 520 with the board 130, the first surface 52a of the barrier wall 52 is positioned more to the Z axis plus side than is the second surface 52b of the barrier wall 52.

The third surface 52c of the barrier wall 52 is a surface formed along the outer edge of the first surface 52a of the barrier wall 52 and the outer edge of the second surface 52b of the barrier wall 52. The third surface 52c of the barrier wall 52 is a surface that includes a surface that is parallel to the YZ plane and a surface that is parallel to the ZX plane. The third surface 52c of the barrier wall 52 is an inner wall surface of the barrier wall 52. The third surface 52c of the barrier wall 52 is a surface that regulates the partition 50.

The fourth surface 52d of the barrier wall 52 is a surface formed along the outer edge of the first surface 52a of the barrier wall 52 and the outer edge of the second surface 52b of the barrier wall 52. The fourth surface 52d of the barrier wall 52 is a surface that includes a surface that is parallel to the YZ plane and a surface that is parallel to the ZX plane. The fourth surface 52d of the barrier wall 52 is a surface that is on the opposite side from the third surface 52c of the barrier wall 52. The fourth surface 52d of the barrier wall 52 is an outer wall surface of the barrier wall 52.

The partitioned member 520 has a protrusion 52e. The partitioned member 520 has the protrusion 52e on the end of the barrier wall 52. The protrusion 52e is a site where a part of the tip portion on the lower edge side, that is, the Z axis minus direction side is formed in a protruding shape. Specifically, the protrusion 52e is a site that protrudes from the second surface 52b of the barrier wall 52. The protrusion 52e is a columnar projection with a diameter smaller than, for example, the thickness of the barrier wall 52. A plurality of protrusions 52e are provided in the barrier wall. A plurality of protrusions 52e are provided in each of the barrier walls, from the first barrier wall 52-1 to the twenty fourth barrier wall 52-24. One protrusion 52e is provided on each side of the barrier walls, from the first barrier wall 52-1 to the twenty fourth barrier wall 52-24. The protrusion 52e functions as a welded part for welding the partitioned member 520 to the board 130.

FIG. 9 is a cross sectional view of the plate 500 that applies the partitioned member 520 of the fourth modified embodiment. Note that the same reference numerals are attached to configuring elements provided by the plate 500 that applies the partitioned member 520 of the fourth modified embodiment as were attached to configuring elements having the same function and configuration that were provided by the plate 100 that applied the partitioned member 120 described in conjunction with FIG. 1 through FIG. 4. Descriptions of configuring elements having the same reference numerals as configuring elements of the partitioned member 120 described in conjunction with FIG. 1 through FIG. 4 are sometimes omitted in the description of the plate 500 that applies the partitioned member 520 of the fourth modified embodiment so as to avoid redundant descriptions.

The plate 500 provides the partitioned member 520, the board 130, and a sealing member 140. The protrusion 52e of the partitioned member 520 abuts with the board 130 in a state where the sealing member 140 is sandwiched between the partitioned member 520 and the board 130, and the location of the abutment is welded. That is, by welding only the protrusion 52e, which is a part of the tip portion on the Z axis minus direction side of the barrier wall 52, to the first surface 130a of the board 130 as the welded part, the partitioned member 520 and the board 130 are joined in a spot like manner. Note that the protrusion 52e can be set in any volume, size or shape. For example, the protrusion 52e may be a protrusion formed in a long thin line along the barrier wall 52 instead of in a cylindrical shape as illustrated in FIG 8. The joint strength between the partitioned member 520 and the board 130 can be adjusted by the shape, size and volume of the protrusion 52e.

The sealing member 140 is a member for sealing a crevice between the partitioned member 520 and the board 130 in a portion where the protrusion 52e has been excluded. The sealing member 140 is configured of an elastic material such as, for example, silicon rubber and the like. The sealing member 140 is configured in, for example, a sheet like form. The sealing member 140 has a hole that corresponds with the bottom shape of a well 510 in a portion corresponding to the bottom of the well 510. The sealing member 140 and the partitioned member 520 are positioned so that the positions of the well 510 and these holes match, and thus the first surface 130a of the board 130 becomes the bottom of the well 510.

The sealing member 140 has a hole that is substantially the same shape and diameter as the protrusion 52e in a position that corresponds to the protrusion 52e. The sealing member 140 is sandwiched between the partitioned member 520 and the board 130 in a state where the protrusion 52e penetrates through the hole.

Note that the sealing member 140 is not limited to a sheet like form. The sealing member 140 may arrange a sealing member shaped like, for example, an O-ring in each well. In this case, the O-ring may be formed so that the protrusion 52e is positioned on the inner circumference of the O-ring or may be formed so that the protrusion 52e is positioned on the outer circumference of the O-ring.

When the sealing member 140 is an elastic member, the height (thickness) of the sealing member 140 is thicker than the protruding height of the protrusion 52e, that is, thicker than the height at which the protrusion 52e protrudes from the second surface 52b of the barrier wall 52. In this case, when the protrusion 52e of the partitioned member 520 abuts with the board 130 through the sealing member 140, the sealing member 140 is in a state of being crushed between the partitioned member 520 and the board 130. In this way, the sealing member 140 can demonstrate good waterproofing performance.

Because the partitioned member 520 and the board 130 are welded in a spot like manner by the protrusion 52e in the plate 500 that applies the partitioned member 520 of the fourth modified embodiment, the joint strength between the partitioned member 520 and the board 130 can be made smaller than in a case where the entire lower end circumference is welded. By this, it is possible, after the partitioned member 520 and the board 130 have been joined by welding, to apply force to the protrusion 52e to separate the partitioned member 520 and the board 130, when necessary. For example, in a configuration where a biochip is supported by the surface of the board 130, as will be described later, the partitioned member is not needed when optical measurement is performed on the biochip. Therefore, the partitioned member is removed from the board before the optical measurement of the biochip is performed to thus enable the optical measurement (observation) of the biochip supported by the board to be performed. By this, an objective lens can be brought closer to the biochip than when, for example, optical measurement of the biochip is performed using a plate where a partitioned member is joined to the board.

Just as with the partitioned member 120, the partitioned member 520 of the fourth modified embodiment has the gap 28 between wells and thus rigidity is low. The partitioned member 520 that suppresses rigidity through the presence of the gap 28 is, in the same way as was described above for the partitioned member 120, deformable when joined and, through that deformation, the protrusion 53e reliably abuts with the board 130. Therefore, it is possible to reduce joint defects in the spot like welding between the partitioned member 520 and the board 130.

The method for manufacturing the plate 500 that applies the partitioned member 520 of the fourth modified embodiment will be described next.

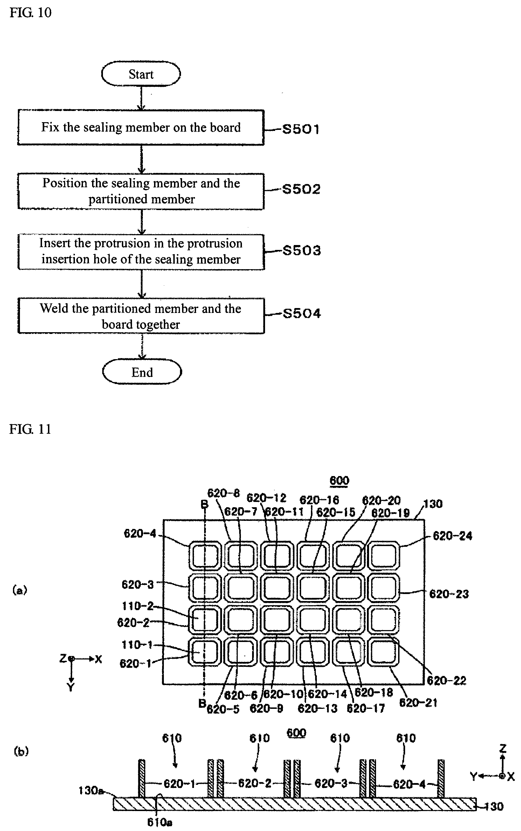

FIG. 10 is a flow chart illustrating the procedure for manufacturing the plate 500.

At step S501, the sealing member 140 is fixed on the first surface 130a of the board 130. Specifically, the sealing member 140 is fixed to the first surface 130a of the board 130 using an adhesive or the like. Note that the sealing member 140 is sandwiched between the board 130 and the partitioned member 520 in a subsequent step and thus may be placed on the first surface 130a of the board 130 without being fixed to the board 130. A state is achieved through step S501 where the board 130 supports the sealing member 140 using the first surface 130a.

A positioning step is executed at step S502. The positioning step positions the partitioned member 520 relative to the sealing member 140, which is supported by the first surface 130a of the board 130. Specifically, the position of the partitioned member 520 is adjusted relative to the sealing member 140 so that the protrusion 52e of the partitioned member 520 aligns with the protrusion insertion hole in the sealing member 140. A state is achieved through step S502 where the partitioned member 520 is positioned relative to the sealing member 140. That is, a state is achieved where the position of the partition 50 of the partitioned member 520 is aligned with the position of the hole provided in the sealing member 140 that corresponds to the well 510.

An inserting step is executed at step S503. The inserting step inserts the protrusion 52e of the partitioned member 520 into the protrusion insertion hole in the sealing member 140. A state is achieved through step S502 where the welded part on the tip of the protrusion 52e of the partitioned member 520 makes contact with the first surface 130a of the board 130.

A welding step is executed at step S504. In the welding step, the partitioned member 520 is welded to the board 130 thus achieving a state where the welded part on the tip of the protrusion 52e of the partitioned member 520 is making contact with the first surface 130a of the board 130. By this, the partitioned member 520 is joined to the board 130. For example, ultrasonic welding can be applied to the welding. Step S504 sandwiches the sealing member 140 between the partitioned member 520 and the board 130, and integrates the partitioned member 520, the board 130 and the sealing member 140 to thus complete the plate 500.

Plate 100, plate 200, plate 300, plate 400, and plate 500 described above were formed by joining one board with one partitioned member, however, plate configuration is not limited to this and thus plates may be formed by joining each of plurality of partitioned members to a single board. For example, when a plate having 24 wells in four rows and six columns like that illustrated in FIG 1 is formed, a plate having 24 wells in four rows and six columns can be configured by joining two partitioned members each having 12 partitions in one board. As an example, a first partitioned member having 12 partitions in four rows and three columns, and a second partitioned member having 12 partitions in four rows and three columns can be used. As another example, a partitioned member having 12 partitions may be, for example, a partitioned member with an L shaped external shape that joins four rows and two columns and two rows and two columns, or may be a partitioned member with a step like external shape that joins four rows and one column, three rows and two columns, and two rows and one column. Furthermore, a plate having 24 wells in four rows and six columns can be configured using, for example, a first partitioned member having four partitions in four rows and one column and a second partitioned member having 20 partitions in four rows and five columns. In this case as well, the partitioned members are not limited to a configuration of four rows and five columns and thus may have L shaped or step like external shapes. Furthermore, a plate having 24 wells in four rows and six columns may be configured by joining three or more partitioned members to a board. A plate having 24 wells can be configured using a first partitioned member having 12 partitions, a second partitioned member having eight partitions, and a third partitioned member having four partitions. Moreover, the shapes of the partitions in the partitioned member can each be different. For example, the shape of the partitions in the first partitioned member may be rectangular while the shape of the partitions in the second partitioned member may be round.

FIG 11 is a drawing a plate 600 that applies a partitioned member 620, which is a fifth modified embodiment of the partitioned member 120. FIG 11 (a) is a top surface view of the partitioned member 620. FIG 11 (b) is a cross sectional view of the partitioned member 620. Note that FIG 11 (b) illustrates a cross section of the plate 600 cut along the cut line B-B in FIG 11 (a). Note that the same reference numerals are attached to configuring elements provided by the plate 600 that applies the partitioned member 620 of the fifth modified embodiment as were attached to configuring elements having the same function and configuration that were provided by the plate 100 that applied the partitioned member 120 described in conjunction with FIG 1 through FIG 5. Descriptions of configuring elements having the same reference numerals as configuring elements of the partitioned member 120 described in conjunction with FIG 1 through FIG 4 are sometimes omitted in the description of the plate 600 that applies the partitioned member 620 of the fifth modified embodiment so as to avoid redundant descriptions.

The plate 600 provides the partitioned member 620 and the board 130.

The partitioned member 620 includes first barrier wall 620-1, second barrier wall 620-2, third barrier wall 620-3, fourth barrier wall 620-4, fifth barrier wall 620-5, sixth barrier wall 620-6, seventh barrier wall 620-7, eighth barrier wall 620-8, ninth barrier wall 620-9, tenth barrier wall 620-10, eleventh barrier wall 620-11, twelfth barrier wall 620-12, thirteenth barrier wall 620-13, fourteenth barrier wall 620-14, fifteenth barrier wall 620-15, sixteenth barrier wall 620-16, seventeenth barrier wall 620-17, eighteenth barrier wall 620-18, nineteenth barrier wall 620-19, twentieth barrier wall 620-20, twenty first barrier wall 620-21, twenty second barrier wall 620-22, twenty third barrier wall 620-23, and twenty fourth barrier wall 620-24. The barrier walls, from the first barrier wall 620-1 through the twenty fourth barrier wall 620-24, are each formed independently. The barrier walls, from the first barrier wall 620-1 through the twenty fourth barrier wall 620-24, are each separate members. The total of 24 barrier walls are arranged in six columns in the long side direction of the board 130 and in four rows in the short side direction of the board 130. Although the first barrier wall 620-1 through the twenty fourth barrier wall 620-24 are all configured as separate bodies, the plate 600 has a total of 24 partitions.

The height (the length in the Z axis direction) of the partitioned member 620, that is, the heights (the lengths in the Z axis direction) of the first barrier wall 620-1 through the twenty fourth barrier wall 620-24, are all the same.

The partitioned member 620 is configured of, for example, a resin. Specifically, the partitioned member 620 is configured of a thermally reversible resin. The partitioned member 620 is, for example, a translucent material. Specifically, the partitioned member 620 is a transparent material. Examples of resins used in the partitioned member 620 include acrylic resins such as poly methyl methacrylate resins and the like, polycarbonate (PC), cycloolefin copolymers (COC), polystyrene (PS) and the like.

It is preferable that the first barrier wall 620-1 through the twenty fourth barrier wall 620-24 all be configured of the same material as the board 130. It is preferable that the partitioned member 620 be configured of the same material as the board 130.

The first barrier wall 620-1 through the twenty fourth barrier wall 620-24 are all joined to the first surface 130a of the board 130. Joining is done using, for example, joining by welding or joining by an adhesive. Ultrasonic welding is an example of joining by welding. The plate 600 has a well 610 formed by joining each of the barrier walls, from the first barrier wall 620-1 through the twenty fourth barrier wall 620-24, to the board 130. The well 610 includes a total of 24 wells formed by joining each of the barrier walls, from the first barrier wall 620-1 through the twenty fourth barrier wall 620-24, to the board 130. The first surface 130a of the board 130 forms the bottom of the wells.

The well 610 supports a biochip. The biochip is supported by a bottom 610a of the well 610, that is, by the first surface 130a of the board 130. The bottom 610a of the well 610 is a support area for supporting the biochip. The bottom 610a of the well 610 is an area the range of which is limited by the partitioned member 620, that is, by each of the barrier walls, from the first barrier wall 620-1 through the twenty fourth barrier wall 620-24. The bottom 610a of the well 610 has a substantially square shape. However, that shape of the bottom 610a of the well 610 is not limited to this and thus may be, for example, a round shape.

The partitioned member 620 can also be perceived as a regulating member for regulating a support area for supporting a biochip. Each of the barrier walls, from the first barrier wall 620-1 through the twenty fourth barrier wall 620-24, can be perceived as a first regulating member through a twenty fourth regulating member.

For example, the first barrier wall 620-1 is a first regulating member that regulates a first support area, and the second barrier wall 620-2 is a second regulating member that regulates a second support area. The first regulating member is a member that is a different body than the second regulating member. The first support area is a different area than the second support area. A first biochip is supported by the first support area, and a second biochip, which is a different chip than the first biochip, is supported by the second support area.

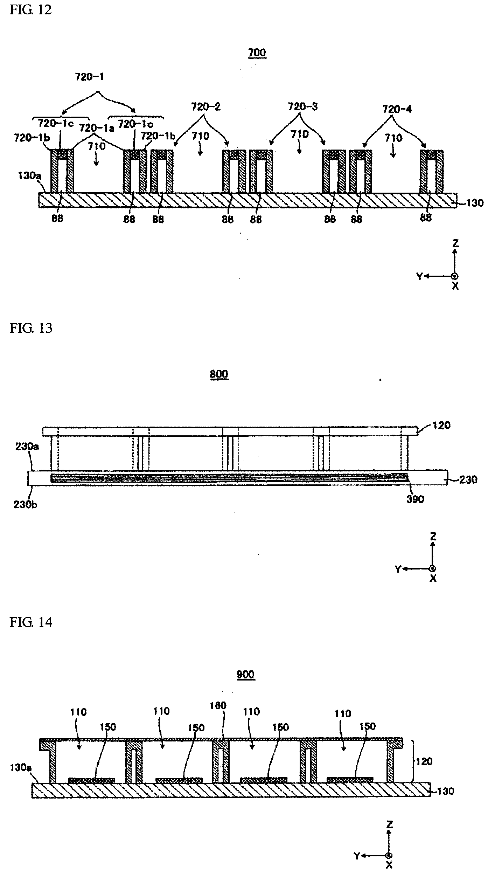

FIG. 12 is a cross sectional view of a plate 700 that applies a partitioned member 720, which is a sixth modified embodiment of the partitioned member 120. Note that the same reference numerals are attached to configuring elements provided by the plate 700 that applies the partitioned member 720 of the sixth modified embodiment as were attached to configuring elements having the same function and configuration that were provided by the plate 100 that applied the partitioned member 120 described in conjunction with FIG. 1 through FIG. 4. Descriptions of configuring elements having the same reference numerals as configuring elements of the partitioned member 120 described in conjunction with FIG 1 through FIG. 4 are sometimes omitted in the description of the plate 700 that applies the partitioned member 720 of the sixth modified embodiment so as to avoid redundant descriptions.

The plate 700 provides the partitioned member 720 and the board 130.

The partitioned member 720 includes first barrier wall 720-1, second barrier wall 720-2, third barrier wall 720-3, fourth barrier wall 720-4, fifth barrier wall 720-5, sixth barrier wall 720-6, seventh barrier wall 720-7, eighth barrier wall 720-8, ninth barrier wall 720-9, tenth barrier wall 720-10, eleventh barrier wall 720-11, twelfth barrier wall 720-12, thirteenth barrier wall 720-13, fourteenth barrier wall 720-14, fifteenth barrier wall 720-15, sixteenth barrier wall 720-16, seventeenth barrier wall 720-17, eighteenth barrier wall 720-18, nineteenth barrier wall 720-19, twentieth barrier wall 720-20, twenty first barrier wall 720-21, twenty second barrier wall 720-22, twenty third barrier wall 720-23, and twenty fourth barrier wall 720-24. The barrier walls, from the first barrier wall 720-1 through the twenty fourth barrier wall 720-24, are each formed independently. The barrier walls, from the first barrier wall 720-1 through the twenty fourth barrier wall 720-24, are each separate members. The total of 24 barrier walls are arranged in six columns in the long side direction of the board 130 and in four rows in the short side direction of the board 130. Although the first barrier wall 720-1 through the twenty fourth barrier wall 720-24 are all configured as separate bodies, the plate 700 has a total of 24 partitions.

The height (the length in the Z axis direction) of the partitioned member 720, that is, the heights (the lengths in the Z axis direction) of the first barrier wall 720-1 through the twenty fourth barrier wall 720-24, are all the same.

The partitioned member 720 is configured of, for example, a resin. Specifically, the partitioned member 720 is configured of a thermally reversible resin. The partitioned member 720 is, for example, a translucent material. Specifically, the partitioned member 720 is a transparent material. Examples of resins used in the partitioned member 720 include acrylic resins such as poly methyl methacrylate resins and the like, polycarbonate (PC), cycloolefin copolymers (COC), polystyrene (PS) and the like.

It is preferable that the first barrier wall 720-1 through the twenty fourth barrier wall 720-24 all be configured of the same material as the board 130. It is preferable that the partitioned member 720 be configured of the same material as the board 130.

The first barrier wall 720-1 through the twenty fourth barrier wall 720-24 are all joined to the first surface 130a of the board 130. Joining is done using, for example, joining by welding or joining by an adhesive. Ultrasonic welding is an example of joining by welding. The plate 700 has a well 710 formed by joining each of the barrier walls, from the first barrier wall 720-1 through the twenty fourth barrier wall 720-24, to the board 130. The well 710 includes a total of 24 wells formed by joining each of the barrier walls, from the first barrier wall 720-1 through the twenty fourth barrier wall 720-24, to the board 130. The first surface 130a of the board 130 forms the bottom of the wells.

The well 710 supports a biochip. The biochip is supported by a bottom 710a of the well 710, that is, by the first surface 130a of the board 130. The bottom 710a of the well 710 is a support area for supporting the biochip. The bottom 710a of the well 710 is an area the range of which is limited by the partitioned member 720, that is, by each of the barrier walls, from the first barrier wall 720-1 through the twenty fourth barrier wall 720-24. The bottom 710a of the well 710 has a substantially square shape. However, that shape of the bottom 710a of the well 710 is not limited to this and thus may be, for example, a round shape.

The partitioned member 720 can also be perceived as a regulating member for regulating a support area for supporting a biochip. Each of the barrier walls, from the first barrier wall 720-1 through the twenty fourth barrier wall 720-24, can be perceived as a first regulating member through a twenty fourth regulating member.

For example, the first barrier wall 720-1 is a first regulating member that regulates a first support area, and the second barrier wall 720-2 is a second regulating member that regulates a second support area. The first regulating member is a member that is a different body than the second regulating member. The first support area is a different area than the second support area. The first support area is a different area than the second support area. A first biochip is supported by the first support area, and a second biochip, which is a different chip than the first biochip, is supported by the second support area.

Each of the barrier walls, from the first barrier wall 720-1 through the twenty fourth barrier wall 720-24, has two walls and a linking part.

This will be described taking the first barrier wall 720-1 as an example.

The first barrier wall 720-1 has a wall 720-1a, a wall 720-1b, and a linking part 720-1c.

The wall 720-1a is a surface that includes a surface that is parallel to the YZ plane and a surface that is parallel to the ZX plane. The wall 720-1a is an interior wall of the first barrier wall 720-1. The wall 720-1a forms a partition.

The wall 720-1b is a surface that includes a surface that is parallel to the YZ plane and a surface that is parallel to the ZX plane. The wall 720-1b is an exterior wall of the first barrier wall 720-1.

The linking part 720-1c links the wall 720-1a and the wall 720-1b. The linking part 720-1c links the wall 720-1a and the wall 720-1b in an upper end (the end on the Z axis plus direction side). The wall 720-1a and the wall 720-1b are separated from one another below the linking part 720c. The wall 720-1a and the wall 720-1b are separated only by the amount interposed by the linking part 720-1c. A gap 88 is a space surrounded by the wall 720-1a, the wall 720-1b, and the linking part 720-1c. The gap 88 becomes a space that is surrounded by the wall 720-1a, the wall 720-1b, the linking part 720-1c, and the board 130 when the partitioned member 720 is joined to the board 130.

The second barrier wall 720-2 through the twenty fourth barrier wall 720-24 all have the same configuration as the first barrier wall 720-1.

Due to the presence of the gap 88, the plate 700 is able to suppress the deformation of the board 130 when the partitioned member 720 is joined to the board 130.

Note that the configuration of the partitioned member 720 is not limited to that illustrated in FIG. 12.

For example, the configuration of the gap described in conjunction with FIG. 5, the configuration of the gap described in conjunction with FIG. 6, and the configuration of the gap described in conjunction with FIG. 7 can all be applied to the partitioned member 720.
For example, as was described in conjunction with FIG. 8 and FIG. 9, the configuration may make the welded part that welds the partitioned member 720 to the board 130 a welded part with a protruding shape, and may provide a sealing member between the partitioned member 720 and the board 130.

A modified embodiment of the board 130 will be described next.

FIG. 13 is a side view of a plate 800 that applies a board 230, which is a modified embodiment of the board 130. Note that the same reference numerals are attached to configuring elements provided by the plate 800 that applies the board 230 of the modified embodiment as were attached to configuring elements having the same function and configuration that were provided by the plate 100 that applied the board 130 described in conjunction with FIG. 1 through FIG. 4. Descriptions of configuring elements having the same reference numerals as configuring elements of the board 130 described in conjunction with FIG. 1 through FIG. 4 are sometimes omitted in the description of the plate 800 that applies the board 230 of the modified embodiment so as to avoid redundant descriptions.

The plate 800 provides the partitioned member 120 and the board 230.

The board 230 is a plate like member. The board 230 has a first surface 230a, a second surface 230b, and a third surface 230c.

The first surface 230a of the board 230 is a surface that is parallel to the XY plane. In order to ensure a desired flatness, the first surface 230a of the board 230 is polished to an accuracy of, for example, several microns.

The second surface 230b of the board 230 is a surface that is parallel to the XY plane. The second surface 230b of the board 230 is formed in a different position in the Z axis direction than the first surface 230a of the board 230. The second surface 230b of the board 230 is parallel to the first surface 230a of the board 230. When the board 230 is joined with the partitioned member 120, the first surface 230a of the board 230 is positioned more to the Z plus side than is the second surface 230b of the board 230. In order to ensure a desired flatness, the second surface 230b of the board 230 is polished to an accuracy of, for example, several microns. When the plate 800 is formed by joining the partitioned member 120 with the board 230, the second surface 230b of the board 230 is a surface that is substantially parallel to the first surface 120a of the partitioned member 120.

The third surface 230c of the board 230 is a surface formed along the outer edge of the first wall 230a of the board 230 and the outer edge of the second surface 230b of the board 230. The third surface 230c of the board 230 is a surface that includes a surface that is parallel to the YZ plane and a surface that is parallel to the ZX plane.

The board 230 has a groove 390.

The groove 390 is formed in the third surface 230c of the board 230. The groove 390 is provided in, for example, each of two surfaces of the third surface 230c of the board that are the surfaces that are parallel to the YZ plane. The groove 390 includes a plurality of grooves. Each of the plurality of grooves is formed along the Y direction.

The board 230 that has the groove 390 has a larger friction coefficient in the Z axis direction than a board that does not have a groove. Therefore, for example, a transport device such as a robotic arm and the like can stably transport the plate 800 by gripping the groove 390 of the plate 800.

The board 230 is configured of, for example, a resin. Specifically, the board 230 is configured of a thermally reversible resin. The board 230 is, for example, a translucent material. Specifically, the board 230 is a transparent material. Examples of resins used in the board 230 include acrylic resins such as poly methyl methacrylate resins and the like, polycarbonate (PC), cycloolefin copolymers (COC), and polystyrene (PS).

It is preferable that the plate 800 be configured of the same material as the partitioned member 120 and the board 230.

Note that while the plate 800 was configured from the partitioned member 120 and board 230, any of the partitioned members, including the partitioned member 220 described in conjunction with FIG 5, the partitioned member 320 described in conjunction with FIG. 6, the partitioned member 420 described in conjunction with FIG 7, the partitioned member 520 described in conjunction with FIG. 8 and FIG. 9, the partitioned member 620 described in conjunction with FIG 11, and the partitioned member 720 described in conjunction with FIG. 12, may be applied instead of the partitioned member 120.

A second embodiment of the present invention will be described next. The second embodiment relates to a plate having a biochip.

FIG 14 is a cross sectional view of a plate 900 according to the second embodiment of the present invention. The plate 900 provides the partitioned member 120, the board 130, a biochip 150, and a protective member 160.

A protective member 160 for the biochip is provided that covers an opening in the top surface side of the well 110.

The biochip 150 is a chip where biomolecules of DNA, proteins and the like have been fixed on a substrate. The biochip 150 is supported by the first surface 130a of the board 130. Specifically, the biochip 150 is fixed to the first surface 130a of the board 130. More specifically, the biochip 150 is fixed to the first surface 130a of the board 130 using an adhesive such as a photocurable adhesive and the like. In this way, the biochip 150 is held inside the well 110.

The protective member 160 protects the well 110. Specifically, the protective member 160 protects the biochip 150 held inside the well 110 from what is outside the well 110. More specifically, the protective member 160 protects the biochip 150 held inside the well 110 from what is outside the well 110 by covering the opening in the well 110. By this, the biochip 150 is protected by the protective member 160 from becoming contaminated before the biochip 150 is used. The protective member 160 is configured of, for example, a film.

When dispensed, a specimen, which is a sample to be measured, is dispensed into the well 110 using a dispensing burette. When the protective member 160 is configured of a film, dispensing can be performed by breaking through the protective member 160 using the nozzle of the dispensing burette and the like. Therefore, the biochip 150 can be kept in a clean state until just before dispensing. After dispensing, the biomolecules on the biochip 150 and targets included in the specimen can be caused to react with one another by shaking the plate 900. When this reaction process is complete, the protective member 160 is removed from the plate 900, and the plate 900 is cleaned to remove any unnecessary specimen. After that, fluorescence measurement and the like is executed in relation to the biochip 150 in the well 110.

Note that the plate 900 may be configured without the protective member 160.

Note that the biochip and the protective member can be combined in the plate 500 (FIG 9) that applied the partitioned member 520 according to the fourth modified embodiment described above. In this case, the protective member is not peeled from the well 110 (or the partitioned member 520), the protective member can be removed together with the partitioned member 520 by separating the partitioned member 520 from the board 130.

The method for manufacturing the plate 900 according to the second embodiment will be described next.

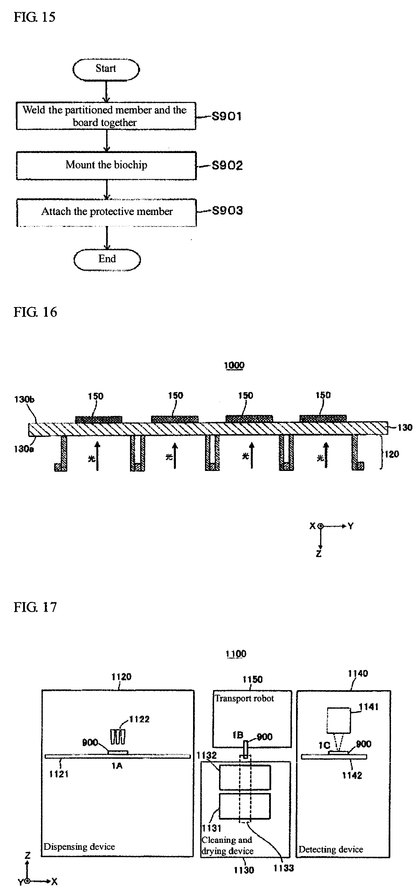

FIG. 15 is a flow chart illustrating the procedure for manufacturing the plate 900.

A welding step is executed at step S901. In the welding step, the entire lower end circumference of each barrier wall 22, which is the welded part 22b of the partitioned member 120, is welded to the first surface 130a of the board 130. By this, the partitioned member 120 is joined to the board 130. For example, ultrasonic welding can be applied to the welding.

At step S902, the biochip 150 is supported by the first surface 130a of the board 130. The biochip 150 is placed on the first surface 130a of the board 130, and is then fixed to the first surface 130a of the board 130 using an adhesive or the like. As an example, the biochip 150 is placed on the first surface 130a of the board 130 using a photocurable adhesive, and is then fixed to the first surface 130a of the board 130 by irradiating light from the second surface 130b of the board 130 to cure the photocurable adhesive.

At step S903, the protective member 160 is attached. The protective member 160 is attached to the first surface 120a of the partitioned member 120 so as to cover the openings in the wells 110 that support the biochips 150. In attaching the protective member 160, it is fitting to use, for example, an adhesive that has a degree of adhesive force that allows the protective member 160 to be subsequently peeled from the partitioned member 120.

The plate 900 having the biochip 150 is thus completed as described above. Note that the manufacturing method described in conjunction with FIG. 15 can suitably be applied, in the same way, as the method for manufacturing a plate 1000, which will be explained next.

FIG. 16 is a cross sectional view of the plate 1000 according to a modified embodiment of the second embodiment of the present invention. The plate 1000 provides the partitioned member 120, the board 130, and the biochip 150. The plate 1000 differs from the plate 900 in that the biochip 150 is attached to the second surface 130b of the board 130 and not to the first surface 130a of the board 130.

In the board 130, the partitioned member 120 is joined to the first surface 130a. In the board 130, the biochip 150 is on the second surface 130b, which is the surface on the opposite side from the first surface 130a. The biochip 150 is supported in a position on the second surface 130b of the board 130 that corresponds to the partition 20 of the partitioned member 120 that is joined to the first surface 130a of the board 130.

The biochip 150 is fixed to the board 130 using, for example, a photocurable adhesive. The biochip 150 is placed on the second surface 130b of the board 130 in a state where the photocurable adhesive has been applied to the back surface (the surface fixed to the board 130) of the biochip 150. The board 130 is a translucent material. The biochip 150 is fixed to the second surface 130b of the board 130 by irradiating light from the first surface 130a of the board 130 to cure the photocurable adhesive. Here, the partitioned member 120 is configured of a material that is not translucent. By this, the barrier wall 22 of the partitioned member 120 plays the role of making it so that light is selectively irradiated toward the back surface of the biochip 150 on which the photocurable adhesive has been applied.

Note that when a specimen is made to react with the biochip 150 of the plate 1000, a method can be used that dispenses the specimen into partitions that correspond to each biochip 150 formed by the partitioned member after setting another partitioned member, that partitions each of the biochips 150, on the second surface 130b of the board 130.

Any of the partitioned members, including the partitioned member 220 described in conjunction with FIG 5, the partitioned member 320 described in conjunction with FIG 6, the partitioned member 420 described in conjunction with FIG. 7, the partitioned member 520 described in conjunction with FIG. 8 and FIG. 9, the partitioned member 620 described in conjunction with FIG. 11, and the partitioned member 720 described in conjunction with FIG 12, may be applied instead of the partitioned member 120 in the plate 900 and the plate 1000 described as the second embodiment, and, furthermore, the board 230 described in conjunction with FIG 13 may be applied instead of the board 130. Furthermore, modified embodiments of the partitioned member and modified embodiments of the board may be suitably combined. Moreover, plates according to these modified embodiments can be manufactured by applying the same method as the method for manufacturing the plate 900 described in FIG 15.

FIG. 17 is a schematic view of a screening device 1100 for executing a screening process for the plate 900 described above, which has a biochip. In FIG. 17, the Z direction is a vertical direction and the XY plane is a horizontal plane. The screening device 1100 is configured by providing a dispensing device 1120, a cleaning and drying device 1130, a detecting device 1140, and a transport robot 1150. The three devices - the dispensing device 1120, the cleaning and drying device 1130, and the detecting device - are arranged side by side in this order in the X axis direction.

The dispensing device 1120 is a device for dispensing relative to the plate 900. As was described above, a plurality of wells are provided in the plate 900, and a biochip is attached to the bottom of each well. The dispensing device 1120 provides a transport stage 1121 that moves in a horizontal direction (the X direction), and a dispensing nozzle 1122 that is arranged above the transport stage 1121. The plate 900 is arranged on the transport stage 1121 in a horizontal state, and is transported by the transport stage 1121 to a specified position under (illustrated by 1A in FIG 17) the dispensing nozzle 1122. A horizontal state means a state where the main surface of the plate 900 is made parallel to a horizontal plane and the opening in the well is facing directly upward. In the specified position described above, a liquid specimen is dispensed into each well of the plate 900 from the dispensing nozzle 1122. The dispensing nozzle 1122, as illustrated in FIG. 17 for example, can be configured by providing the same number of nozzles in a linear fashion as one row's worth of wells of the plate 900 at the same intervals as the wells. When this type of linear dispensing nozzle is used, a method can be used that dispenses for a row's worth of wells all at once and then proceeds, in order, to dispense in the neighboring row. Furthermore, a configuration may be used that uses the same number of nozzles as the total number of wells of the plate 900 provided in a lattice pattern in the same arrangement as the wells and thus dispenses in all of the wells of the plate 900 at once. Furthermore, a configuration may be used that uses a dispensing nozzle that has just one nozzle and thus dispenses in one well at a time in order.

Specific biomolecules that react specifically with a variety of target molecules (hereinafter referred to as targets) that may be included in a specimen are fixed on the surface of the biochip. In order to accelerate a reaction between these biomolecules and the targets in the specimen, the transport stage 1121 may shake the plate 900 and heat the plate 900 to a temperature suitable for promoting a good reaction. After that, the plate 900 is transported by the transport stage 1121 to a position (on the downstream side) that is close to the cleaning and drying device 1130.

The cleaning and drying device 1130 is the device that cleans and dries the plate 900 after dispensing in preparation for the processing done by the detecting device 1140. The cleaning and drying device 1130 provides a cleaning unit 1131, a drying unit 1132 and a transport unit 1133, and the drying unit 1132 is arranged above (in the + Z direction) the cleaning unit 1131. For example, as illustrated in the figure, the cleaning and drying device 1130 is configured so that the cleaning unit 1131 is on the bottom and the drying unit 1132 is on the top in a position that is lower than the horizontal height of the transport stage 1121 of the dispensing unit 1120. After dispensing, the plate 900 that has been transported downstream from the dispensing device 1120 is gripped by a robotic arm (not illustrated in the figure) of a transport robot 1150 set, for example, above (in the + Z direction) the cleaning and drying device 1130 and handed to the cleaning and drying device 1130. At this time, the robotic arm of the transport robot 1150 grasps the plate 900 placed on the transport stage 1121 of the dispensing device 1120, rotates the gripped plate 900 to an upright state, and then sets the plate 900 in the upright state in the transport unit 1133 of the cleaning and drying device 1130 (1B in FIG. 17). An upright state means a state where the main surface of the plate 900 is made perpendicular to the horizontal plane and the opening in the well is facing horizontally. In the example in FIG 17, the plate 900 is rotated 90° clockwise or counterclockwise, with the Y axis in FIG. 17 as the rotational axis, from the horizontal state illustrated by 1A in FIG 17 to the upright state illustrated by 1B in FIG 17. That is, the plate 900 is in a state where the opening of the well is facing in the +X (right) or the -X (left) direction in FIG. 17.

First, the cleaning and drying device 1130 transports the plate 900 in the upright state set in the transport unit 1133 down (in the -Z direction) to the cleaning unit 1131, and the plate is cleaned in the cleaning unit 1131; next, the device transports the plate up (in the + Z direction) to the drying unit 1132, and the plate is dried in the drying unit 1132. While the plate 900 initially passes through the drying unit 1132 when being transported down, the drying unit 1132 is not running at the time. The plate 900 is cleaned and dried in the order described above while being held in the upright state by the transport unit 1133, and is then transported by the transport unit 1133 to above (the position illustrated by 1B in FIG. 17) the cleaning and drying device 1130.

The detecting device 1140 is a device for performing a measurement in order to detect the affinity between the biomolecules and the targets described above in relation to the biochip on the cleaned plate 900. The detecting device 1140 provides an imaging device 1141 that images the biochip to generate an inspection image, and an inspection stage 1142 that places the plate 900 during imaging. The imaging device 1141 has a configuration that includes a light source, an optical system (a lens and the like), and a camera. As described above, the plate 900 transported to above the cleaning and drying device 1130 after cleaning and drying is gripped by the robotic arm of the transport robot 1150 and is handed to the detecting device 1140. At this time, the robotic arm of the transport robot 1150 grasps the plate 900 being held in the upright state in the transport unit 1133 of the cleaning and drying device 1130, rotates the plate 900 90° to the horizontal state, and then mounts the plate on the inspection stage 1142 of the detecting device 1140. The plate 900 mounted on the inspection stage 1120 is transported to the imaging area (1C in FIG. 17) of the imaging device 1141 by the inspection stage 1142 and is measured.

Measurement by the detecting device 1140 can be performed, for example, by imaging the fluorescence generated by the biomolecules on the biochip using the camera of the imaging device 1141. Only locations of the biomolecules on the biochip that specifically react with the targets in the specimen generate fluorescence when light from the light source is irradiated. Therefore, an inspection image obtained by imaging the biochip is an image where positions corresponding to biomolecules that have reacted to the targets are made bright through fluorescence. By image analyzing this image, or rather, the position and brightness of the fluorescence, it is possible to detect which biomolecules on the biochip the targets in the specimen have an affinity for.

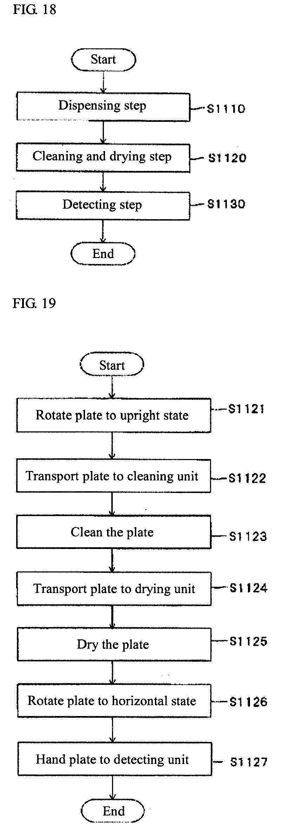

FIG. 18 is a flow chart illustrating a screening method using a screening device 1100.

First, a dispensing step is executed in the dispensing device (step S1110). For example, an operator, who is the operator of the screening device 1100, sets the plate 900 to be inspected in the transport stage 1121 of the dispensing device 1120, and then starts the dispensing step by inputting a start command into a computer (not illustrated in the figure) that controls the operation of the screening device 1100. When the dispensing step begins, the dispensing device 1120 transports the set plate 900 to a specified dispensing position using the transport stage 1121 in accordance with the control from the control computer, and the specimen is dispensed into each of the wells in the plate 900 from the dispensing nozzle 1122. After dispensing, the transport stage 1121 transports the plate 900 downstream.

Next, a cleaning and drying step is executed in the cleaning and drying device 1130 (step S1120). This step is described later. Note that when the plate to be inspected has a structure where the partitioned member 520 and the board 130 can be separated again as in the plate 500 according to the fourth modified embodiment of the first embodiment, the operator may, for example, remove the partitioned member 520 from the plate 500 by executing a removing step using a manual operation after the cleaning and drying step.

When the cleaning and drying step finishes, a detecting step is then executed in the detecting device 1140 (step S1130). The detecting device 1140 receives the plate 900 that has been cleaned and dried by the cleaning and drying step from the transport robot 1150, and then measures the biochip on the plate 900 in accordance with the control of the control computer. Specifically, the detecting device 1140 images each of the biochips 150 on the plate 900 using the imaging device 1141 to generate inspection images of each of the biochips 150. Furthermore, the detecting device 1140 detects the affinity between the biomolecules on the biochip 150 and the targets contained in the specimen. The inspection results can be stored as electronic data in, for example, a storage unit inside the control computer. Note that the operator may execute an observation step by, for example, observing the captured image on the monitor screen of the image device 1141, directly observing the biochip 150 under a microscope, and the like.

FIG. 19 is a flowchart illustrating a cleaning and drying step in the cleaning and drying device 1130.

First, the transport robot 1150, by driving the robotic arm in accordance with the control of the control computer, grasps the plate 900 placed on the transport stage 1121 of the dispensing device 1120 in the horizontal state with the robotic arm and then rotates the gripped plate 900 90° into the upright state (step S1121). Furthermore, the transport robot 1150 drives the robotic arm in accordance with the control of the control computer to set the plate 900 in the upright state in the transport unit 1133 of the cleaning and drying device 1130.

Next, the cleaning and drying device 1130, by driving the transport unit 1133 in accordance with the control of the control computer, transports the plate 900 set in the transport unit 1133 down to the cleaning unit 1131 (step S1122). Note that, at this time, the control computer controls the drying unit 1132 in a driving stop state when the plate 900 initially passes through the drying unit 1132 so that dry air does not hit the plate 900 before cleaning.

Next, the cleaning and drying device 1130, by driving the cleaning unit 1131 in accordance with the control of the control computer, executes a cleaning step in relation to the plate 900 (step S1123). The cleaning unit 1131 executes cleaning of the plate 900 using a suitable method that atomizes and then sprays, or intermittently sprays and the like, a cleaning solution from a cleaning nozzle (not illustrated in the figure). Furthermore, for example, the control computer may, in addition to performing spray control for the cleaning solution in relation to the cleaning unit 1131, also spread the cleaning solution uniformly over the entire plate 900 so that cleaning residue does not occur by controlling the transport unit 1133 so that the plate 900 moves up and down.

Next, the cleaning and drying device 1130, by driving the transport unit 1133 in accordance with the control of the control computer, transports the cleaned plate 900 up to the drying unit 1132 (step S1124).

Next, the cleaning and drying device 1130, by driving the drying unit 1132 in accordance with the control of the control computer, executes a drying step in relation to the plate 900 (step S 1125). After drying, the transport unit 1133, in accordance with the control of the control computer, transports the plate 900 to the top surface of the cleaning and drying device 1130.

Next, the transport robot 1150, by driving the robotic arm in accordance with the control of the control computer, grasps the plate 900, which has finished drying and is held in the transport unit of the cleaning and drying device 1130, in the upright state, and then rotates the gripped plate 900 90° into the horizontal state (step S1126).

Next, the transport robot 1150 drives the robotic arm in accordance with the control of the control computer to hand the plate 900 in the horizontal state to the detecting device 1140 (step S 1127).

Note that while the screening device 1100 and a screening method having the plate 900 as a target were described, this screen device 1100 and screening method can be applied in the same way to any plate provided with the biochip 150 according to the second embodiment.

While the embodiments of the present invention were described above, the present invention is not limited thereto and thus a variety of changes are possible within a scope that does not depart from the gist of the present invention.

Furthermore, a plate according to another embodiment of the present invention may be a multi well plate that provides a well array (partitioned member) formed by aligning a plurality of wells having openings in two opposing surfaces, and a transparent bottom plate (board) attached to the array so as to form a common bottom in each well on one opening side of the wells, where each of the wells of the well array is formed as a space partitioned using a well wall (barrier wall) that surrounds the well, and each of the well walls is linked to the well wall of the neighboring well on the other opening side of the well and separated from another well wall in the bottom surface side of the well.

Furthermore, the bottom plate of the well array may be ultrasonically welded in the multi well plate.

Furthermore, the bottom plate of the well array may be ultrasonically welded across the entire circumference of the well wall in the multi well plate.

Furthermore, an elastic sealing material that is sandwiched between the bottom plate and the well wall is provided in the multi well plate, and the well array and the bottom plate may be ultrasonically welded in a spot like manner on the circumference of the well wall.

Moreover, a biochip fixed on the bottom inside the well and a protective cover for the biochip that covers the other opening of the well may be provided in the multi well plate.

### (Reference Numerals)

22 Barrier wall
22f Welded part
24 Linking part
26 Reinforcing part
28 Gap
35 Welded part
100, 200, 300, 400, 500, 600, 700, 800, 900, 1000 Plate
110 Well
120, 220, 320, 420, 520, 620, 720 Partitioned member
130,230 Board
140 Sealing member
150 Biochip
390 Groove
1100 Screening device
1120 Dispensing device
1130 Cleaning and drying device
1140 Detecting device

## Claims

1. A plate, comprising:
a board having a first surface; and
a partitioned member that forms a plurality of partitions using barrier walls,
wherein a gap, which is a space between adjacent barrier walls, is formed on the first surface side,
an end on the gap side of the barrier wall has a welded part that joins with the first surface of the board, and the board and the partition member are welded together at the welded part.

2. The plate according to claim 1, wherein the board and partitioned member above are joined together using ultrasonic welding.

3. The plate according to claim 1 or 2, wherein the welded part is welded to the first surface of the board across the entire circumference of the barrier wall.

4. The plate according to claim 1 or 2, wherein the welded part is welded to the first surface of the board on a portion of the circumference of the barrier wall.

5. The plate according to claim 4, wherein the welded part has a protruding shape, and a sealing member is provided arranged between the partitioned member and the board.

6. The plate according to claim 5, wherein the protruding welded part passes through the sealing member.

7. The plate according to any one of claims 1 through 6, wherein the board and the partitioned member are formed using the same optically transparent resin material.

8. The plate according to any one of claims 1 through 7, wherein a protective film that covers the open surface of the partition is provided.

9. The plate according to any one of the claims 1 through 8, wherein a biochip is provided that is arranged in the bottom of the partition in the first surface.

10. A method for manufacturing a plate having a plurality of wells formed by joining a partitioned member in a first surface of a board, wherein a barrier wall of the partitioned member that forms the wells has a gap, which is a space between adjacent barrier walls, on the first surface side of the board, comprising:
a welding step that welds the first surface of the board and the partitioned member together at a welded part provided on an end on the gap side of the barrier wall.

11. The method for manufacturing a plate according to claim 10, wherein, in the welding step, the welded part of the partitioned member and the first surface of the board are brought into contact and joined by heating past a melting point and then applying pressure.

12. The method for manufacturing a plate according to claim 10 or 11, wherein, in the welding step, the welded part of the partitioned member and the first surface of the board are ultrasonically welded together.

13. The method for manufacturing a plate according to any one of claims 10 through 12, wherein a sealing member having a shape that follows the shape of an end on the gap side of the barrier wall on the first surface of the board is provided, comprising:
a step that aligns the positions of the sealing member and the partitioned member.

14. The method for manufacturing a plate according to any one of claims 10 through 13, wherein, in the welding step, the welded part is welded across the entire circumference of the barrier wall against the first surface of the board.

15. The method for manufacturing a plate according to any one of claims 10 through 13, wherein, in the welding step, the welded part is welded on a portion of the circumference of the barrier wall against the first surface of the board.

16. The method for manufacturing a plate according to claim 13, comprising:
an inserting step that inserts the welded part into the sealing member and then brings the first surface of the board and the welded part of the partitioned member into contact.

17. A method for observing a biochip, comprising:
a removing step that removes the partitioned member from the plate; and
an observing step for observing a biochip arranged on the first surface of the board.

18. A screening method that uses a plate having a biochip, comprising:
a dispensing step for dispensing a specimen containing a target that is able to react specifically to a biomolecule fixed on the biochip in the plate according to claim 9;
a cleaning step for cleaning the plate;
a drying step for drying the plate; and
a detecting step for detecting affinity between the target and the biomolecule.

19. A plate, comprising:
a board; and
a regulating member that is joined to the board through welding and regulates a support area in order to support the biochip.

20. A plate, in the plate according to claim 19, comprising:
a first regulating member that regulates a first support area in order to support a first biochip where the regulating member is joined to the board through welding; and
a second regulating member that regulates a second support area in order to support a second biochip, that is a member configured with a body that is separate from the first regulating member, and is joined to the board through welding.

21. A plate, in the plate according to claim 20, wherein the length of the first regulating member and the length of the second regulating member are the same in a direction perpendicular to the surface where the board and the regulating members join.

22. A plate, in the plate according to any one of claims 19 through 21,
wherein the regulating member has a gap on the end that joins with the board.

23. A plate, in the plate according to any one of claims 19 through 22, wherein the support areas regulated by the regulating members are round.

24. A plate, in the plate according to any one of claims 19 through 23, wherein the board is formed using a material that is translucent.

25. A plate, in the plate according to any one of claims 19 through 24, wherein the regulating members are formed using a material that is translucent.

26. A plate, in the plate according to any one of claims 19 through 25, wherein the board is formed from the same material as the regulating members.

27. A plate, in the plate according to any one of claims 19 through 26, wherein the regulating members are joined to the board using ultrasonic welding.
